# EUROPEAN PATENT SPECIFICATION

(11) **EP 2 370 021 B1**
(45) Date of publication and mention of the grant of the patent: **06.09.2017**
(21) Application number: 09828728.7
(22) Date of filing: 25.11.2009
(51) Int. Cl.: A61F 2/02, A61M 13/00

(54) **APPARATUS FOR TRANSPORTATION OF OXYGEN TO IMPLANTED CELLS**
VORRICHTUNG FÜR DEN TRANSPORT VON SAUERSTOFF IN IMPLANTIERTE ZELLEN
APPAREIL PERMETTANT LE TRANSPORT D'OXYGÈNE À DESTINATION DE CELLULES IMPLANTÉES

(30) Priority: 26.11.2008 US 315102
(43) Date of publication of application: 05.10.2011
(73) Proprietor: Beta O2 Technologies Ltd., 49511 Petach Tikva (IL)
(72) Inventor: STERN, Yaki, 73142 Shoham (IL); BARKAI, Uriel, 30815 MP Hof-Carmel (IL); ROTEM, Avi, 49202 Petach Tikva (IL); REINGEWIRTZ, Meir, 47205 Ramat Hasharon (IL); ROZY, Yoram, 38900 Caesarea (IL)
(74) Representative: Hirsch & Associés
(86) International application number: PCT/IL2009/001114
(87) International publication number: WO 2010/061387

(56) References cited:
- WO-A2-2008/079997
- WO-A2-2009/031154
- US-A1- 2003 050 622
- US-A1- 2003 087 427
- US-A1- 2004 133 188
- US-A1- 2008 086 042
- US-A1- 2009 012 502

## Description

### FIELD OF THE INVENTION

Some applications of the present invention relate generally to implantable medical devices. Specifically, some applications of the present invention relate to an implantable device to generate and provide oxygen to transplanted cells, e.g., cells in transplanted pancreatic islets.

### BACKGROUND

Oxygen is vital to many physiological and metabolic processes, including aerobic metabolism. An insufficient supply of oxygen to implanted cells often leads to cell injury or death. Oxygen provision is a vital component in sustaining transplanted cells.

In healthy individuals, insulin release is regulated so as to maintain blood glucose levels in the range of about 70 to 110 milligrams per deciliter. In diabetics, insulin is either not produced at all (Type I diabetes), or the body cells do not properly respond to the insulin that is produced (Type II diabetes). The result is elevated blood glucose levels.

The success of many cellular transplants is compromised not only due to graft-host rejections, but also on account of ischemic conditions generated by insufficient oxygen supply to the transplant. Following implantation of the cells, oxygen is provided to the implanted cells from the body tissue (mainly, via diffusion), and in some cases, from vascular structures that form around the transplanted cells with the help of angiogenic factors, e.g., VEGF and bFGF. However, the natural diffusion rate is too low to provide the cells with a significant, necessary amount of oxygen.

PCT Publication WO 01/50983 to Vardi et al., and US Patent Application Ser. No. 10/466,069 in the national phase thereof describe an implantable device comprising a chamber for holding functional cells and an oxygen generator for providing oxygen to the functional cells. In one embodiment, the oxygen generator is described as comprising photosynthetic cells that convert carbon dioxide to oxygen when illuminated. In another embodiment, the oxygen generator is described as comprising electrodes that produce oxygen by electrolysis.

US Patent Application Publication 2005/0136092 to Rotem, which is incorporated herein by reference, describes apparatus including a chamber, which is adapted to be implanted in a body of an individual, the chamber including functional cells and chlorophyll-containing elements comprising chlorophyll of an obligate photoautotroph. Typically, the chlorophyll-containing elements include intact photosynthetic cells and/or isolated chloroplasts. The chlorophyll-containing elements provide oxygen to the functional cells and/or consume carbon dioxide produced by the functional cells. The chamber has one or more walls that are adapted to be permeable to nutrients and substances produced or secreted by the cells. The walls also typically immunoisolate the cells from constituents of the body. The chamber is adapted to be implanted under skin of the subject, or in the peritoneum. The apparatus further comprises a light source that is adapted to provide light to the chlorophyll-containing elements. The chamber may comprise an oxygen sensor that detects an oxygen concentration in a vicinity of the functional cells, and/or in a vicinity of the chlorophyll-containing elements. Providing the light in the series of pulses generally reduces power consumption of the apparatus, and/or provides control of the quantity of oxygen produced by the chlorophyll-containing elements, and/or provides control of the quantity of carbon dioxide consumed by the chlorophyll-containing elements. In some embodiments of the invention, the chamber comprises an oxygen reservoir, which typically comprises a material that stores and releases oxygen, such as responsively to an oxygen concentration in a vicinity of the reservoir. The oxygen reservoir typically stores oxygen produced by the chlorophyll-containing elements that is in excess of the current needs of the functional cells, and releases the stored oxygen if insufficient oxygen is later generated by the chlorophyll-containing elements.

PCT Publication WO 06/059322 to Evron et al. describes apparatus including a chamber which is adapted to be implanted in a body of an individual. The chamber includes functional cells and chlorophyll-containing elements comprising chlorophyll of an obligate photoautotroph. Other embodiments are also described.

US Patent 5,713,888 to Neuenfeldt et al. describes an implant assembly for a host tissue. The implant assembly comprises a pouch including wall means defining a chamber for holding a second member. The wall means includes an outer vascularizing membrane having a conformation that results in growth of vascular structures by the host tissue, close to an interface between the vascularizing membrane and host tissue. The assembly includes a second member that can be removably inserted in the chamber including an interior for receiving cells and wall means defining an immuno-isolating membrane that isolates the cells from the immune response of the host tissue.

US Patent 6,368,592 to Colton et al. describes techniques for supplying oxygen to cells in vitro or in vivo by generating oxygen with an oxygen generator that electrolyzes water to oxygen and hydrogen. The oxygen generator is described as supplying oxygen to cells contained in an encapsulating chamber for implanting in the body, such as an immunoisolation chamber bounded by a semipermeable barrier layer that allows selected components to enter and leave the chamber. A bioactive molecule may be present with the cells.

US Patent 6,960,351 to Dionne et al. describes an immunoisolatory vehicle for the implantation into an individual of cells which produce a needed product or provide a needed metabolic function. The vehicle is comprised of a core region containing isolated cells and materials sufficient to maintain the cells, and a permselective, biocompatible, peripheral region free of the isolated cells, which immunoisolates the core yet provides for the delivery of the secreted product or metabolic function to the individual. The vehicle is described as being particularly well-suited to delivery of insulin from immunoisolated islets of Langerhans, and as being used advantageously for delivery of high molecular weight products, such as products larger than IgG. A method of making a biocompatible, immunoisolatory implantable vehicle is also described, consisting in a first embodiment of a coextrusion process, and in a second embodiment of a stepwise process. A method is described for isolating cells within a biocompatible, immunoisolatory implantable vehicle, which protects the isolated cells from attack by the immune system of an individual in whom the vehicle is implanted. A method is described of providing a needed biological product or metabolic function to an individual, comprising implanting into the individual an immunoisolatory vehicle containing isolated cells which produce the product or provide the metabolic function.

The '351 patent describes a vehicle that provides, in at least one dimension, sufficiently close proximity of any isolated cells in the core to the surrounding tissues of the recipient, including the recipient's bloodstream, in order to maintain the viability and function of the isolated cells. However, the diffusional limitations of the materials used to form the vehicle do not in all cases solely prescribe its configurational limits. Certain additives can be used which alter or enhance the diffusional properties, or nutrient or oxygen transport properties, of the basic vehicle. For example, the internal medium can be supplemented with oxygen-saturated perfluorocarbons, thus reducing the needs for immediate contact with blood-borne oxygen. This is described as allowing isolated cells or tissues to remain viable while, for instance, a gradient of angiotensin is released from the vehicle into the surrounding tissues, stimulating ingrowth of capillaries.

U.S. Patent 4,721,677 to Clark, Jr. et al. describes an implantable biosensor and method for sensing products, such as hydrogen peroxide, generated from an enzymatic reaction between an analyte, like glucose, and an enzyme in the presence of oxygen. The biosensor is described as being equipped with an enclosed chamber for containing oxygen, and can be adapted for extracting oxygen from animal tissue adjacent the container. The biosensor is designed to optically or electrically sense products generated from the enzymatic reaction, which serve as a function of the analyte.

References and methods for use of perfluorocarbons are described in Faithful, N. S. Anaesthesia, 42, pp. 234-242 (1987) and NASA Tech Briefs MSC-21480, U.S. Govt. Printing Office, Washington, D.C. 20402.

US Patent Application Publication 2005/0025680 to Monzyk et al. describes a photolytic cell and a photolytic artificial lung incorporating such a cell. The photolytic artificial lung converts water to oxygen for blood absorption, regulates pH, removes carbon dioxide, and co-produces electrical power. The photolytic artificial lung includes a photolytic cell where all of the chemical reactions occur. Additionally, Monzyk describes photolytically-sensitive materials for oxygen generation. These materials are useful for gas-free artificial lung fabrication. The photolytic cell is described as being useful for directing chemical reactions in organs other than the lung, and for maintaining breathing air in confined systems.

The following patents and patent applications may be of interest:
PCT Publication WO 01/050983 to Vardi
PCT Publication WO 07/138590 to Gross
PCT Publication WO 08/062417 to Rotem
PCT Publication WO 08/065660 to Rotem
PCT Publication WO 09/031154 to Stern
US Patent 2,564,977 to Hu
US Patent 5,614,378 to Yang et al.
US Patent 6,268,161 to Han, et al.
US Patent 6,383,478 to Prokop, et al.
US Patent 6,630,154 to Fraker, et al.
US Patent 6,815,186 to Clark, Jr. et al.
US Patent Application Publication 2003/0113302 to Revazova et al.
US Patent Application Publication 2004/0133188 to Vardi
US Patent Application Publication 2004/0178358 to Kreiss
US Patent Application Publication 2006/0024276 to Ricordi et al.
US Patent Application Publication 2006/0035370 to Lee
US Patent Application Publication 2009/0012502 to Rotem

The following articles may be of interest:
Kaisers U et al., "Liquid ventilation," British Journal of Anaesthesia 91(1):143-151 (2003)
Lacy PE et al., "Maintenance of normoglycemia in diabetic mice by subcutaneous xenografts of encapsulated islets," Science 1782-4 (1991)
Lorch H et al., "Central Venous Access Ports Placed by Interventional Radiologists: Experience with 125 Consecutive Patients," Journal CardioVascular and Interventional Radiology, Pages 180-184, Issue Volume 24, Number 3 (2001)
Silva AI et al., "An overview on the development of a bio-artificial pancreas as a treatment of insulin-dependent diabetes mellitus," Med Res Rev 26(2):181-222 (2006)
Waschke KF and Frietsch T, "Modified haemoglobins and perfluorocarbons" (Current Opinion in Anaesthesiology. 12(2):195-202 (1999)

### SUMMARY OF THE INVENTION

In some applications of the present invention, apparatus comprises a housing for containing transplanted cells that is designated for subcutaneous implantation into the body of a subject. The transplanted cells typically comprise functional cells, e.g., cells disposed in pancreatic islet of Langerhans cells, and in this case are typically in islets. The functional cells are typically disposed in a layer of liquid or gel. The housing comprises an oxygen delivery interface (e.g., a penetrable surface, one or more valves, or one or more tubes) that facilitates the transfer of oxygen to the cells. The apparatus comprises a source of oxygen, or an oxygen supply (e.g., a vessel comprising air, another mixture of gases, or pure oxygen), that is connectable to the housing via the interface. At regular intervals, e.g., every few hours or every few weeks, typically, at least once a week, the subject connects the source of oxygen to the interface, and the source of oxygen supplies a limited amount of oxygen to the housing.

In some applications, the source of oxygen comprises a container comprising a plurality of gases including oxygen. The gases are disposed in the container at a pressure of 1 atm or higher. Typically, the source of oxygen comprises around 5% carbon dioxide in order to maintain a balance of concentrations of carbon dioxide inside the housing and outside the housing. For some applications, the source of oxygen comprises a liquid comprising oxygen carriers (e.g., hemoglobin-based oxygen carriers such as chemically-modified hemoglobin, or "microbubbles" which comprise fluorocarbons such as dodecafluoropentane, perfluorodecalin, or other perfluoro-chemicals) that are loaded with oxygen prior to the injection of the carriers into the housing.

Typically, the housing comprises an oxygen reservoir, which functions as a conduit for oxygen diffusion as well as a reservoir for storing excess oxygen that is supplied to the housing by the source of oxygen. In some applications, the oxygen reservoir comprises a gas reservoir, which is an area of gas in the housing comprising oxygen and carbon dioxide. In some applications, the oxygen reservoir comprises liquid-based oxygen carriers. In such applications, the oxygen carriers function to store, or carry, oxygen when in excess, and release the oxygen upon a need therefor.

For some applications, the housing comprises a plurality of projections which project radially from a center of the housing. The oxygen carriers are typically stored within the projections, which provide increased surface area for absorbing oxygen from vasculature surrounding the housing. The oxygen absorbed via the projections is stored in the housing by the oxygen carriers.

Typically, oxygen is supplied to the housing in a volume and concentration in accordance with the size of the housing and with the number of functional cells disposed therein. Additionally, the amount of oxygen delivered to the housing depends on the composition of the oxygen carriers injected into the housing. That is, a given volume of fluid comprising preloaded oxygen carriers will sustain the functional cells in the housing for a longer period than will the same volume of fluid comprising free oxygen. In general, the oxygen delivery interface facilitates the provision, on a consistent basis, of oxygen to the functional cells in a volume and concentration sufficient to meet the oxygen consumption rate of the functional cells over a given period of time, e.g., between 12 hours and 2 weeks.

In some applications, the oxygen delivery interface comprises a surface of the housing that comprises a material that is penetrable, e.g., rubber, silicone, or plastic, and provides access to an interior of the housing following penetration of the surface. In such applications, the source of oxygen is coupled to a device, e.g., a needle, which transcutaneously accesses the oxygen delivery interface of the housing. For applications in which the source of oxygen is coupled to a needle, the needle punctures the skin of the subject and subsequently the surface of the housing.

Fluid having a low oxygen content disposed within the housing is removed therefrom in conjunction with the supplying to the housing of the fluid having a high oxygen content. (It is noted that "fluid" includes within its scope both liquids and gases.) In some applications, the needle comprises a double-chambered needle having an input chamber and an output chamber. Fluid having a high oxygen content is actively injected into the housing via the input chamber (e.g., by the user operating a normal syringe, or by an electrical mechanism). In conjunction with the injecting, and in response to the pressure introduced within the housing due to the injection of the fluid having a high oxygen content, fluid disposed within the housing is passively withdrawn therefrom through the outlet chamber.

In some applications, the needle comprises a single-chambered needle, and the source of oxygen comprises a syringe that is coupled to a pump. Prior to the supplying of the oxygen to the cells, the syringe is coupled to the needle, the needle punctures the housing, and the pump draws a portion of fluid having a low oxygen content from within the housing through the syringe. A portion of fluid having a high oxygen content within the syringe is then injected into the housing. In such applications, the pump facilitates cycling of (a) active drawing of fluid from within the housing, and (b) the replenishing of the fluid. In some applications, the user performs this cycling, without a pump.

In some applications, the housing is in fluid communication with one or more ports which each comprise a penetrable surface, which facilitates access to the interior of the housing by a needle. The ports are in contact with the subcutaneous tissue of the subject. In some applications, the ports are directly coupled to a surface of the housing that is in contact with subcutaneous tissue. Alternatively, the ports are disposed remotely with respect to the housing and are coupled thereto via respective tubes.

In some applications, the housing is in fluid communication with a fluid inlet tube and a fluid outlet tube having respective first ends thereof that are disposed within the housing. The respective second ends of the input and output tubes are disposed outside the body of the subject. Typically, the end of the input tube that is disposed outside the body of the subject serves as the oxygen delivery interface. Oxygen from the source of oxygen is injected via the input tube and into the housing in order to replenish the fluid and increase the oxygen concentration in the housing. In conjunction with the active injecting of the fluid into the housing, fluid disposed within the housing passively exits the housing via the output tube or is actively drawn from the housing by coupling a source of suction to the outlet tube.

In some applications, the housing is flexible and is implanted in a vicinity of a ribcage of the subject. In such applications, the oxygen-containing fluid is actively, continuously transported within the housing in response to movement of the flexible housing occurring responsively to the natural movements of the ribcage.

In some applications of the present invention, apparatus comprises a housing containing the functional cells that is implanted subcutaneously in a vicinity of a trachea of the subject. The housing is indirectly coupled to the trachea by a fluid transport tube having a first end disposed within the housing, and a second end disposed adjacent to the trachea. A "T"-shaped tracheal mount couples the fluid transport tube to the trachea and serves as the oxygen delivery interface by creating a conduit for supplying air to the housing from the trachea. The housing comprises a depressible upper surface that is in contact with the subcutaneous tissue of the subject. The depressible surface is depressible by the subject who pushes on a portion of his or her skin that is disposed above the depressible surface. By pushing the depressible surface, air is forced out of the housing, through the fluid transport tube, and into the trachea of the subject. The depressible surface is resilient and returns to its original state following the pushing of the surface by the subject. Consequently, reduced pressure is generated in the housing, which draws air from the trachea into the housing, via the fluid transport tube.

For some applications, at least one layer of functional cells, as well as an oxygen generator comprising at least one photosynthetic oxygen supply are disposed within the housing. Typically, the functional cells are disposed in a layer of liquid or gel, such as alginate, agarose, or polyethylene glycol (PEG) and/or dispersed in a three-dimensional biodegradable or non-biodegradable fibrillar matrix. The oxygen generator is typically coupled to a support that is disposed within the housing.

The housing comprises air gaps, e.g., an area of gas comprising oxygen and carbon dioxide, disposed in part between the layers of the system. In some applications, the apparatus comprises one or a plurality of photosynthetic oxygen supplies, e.g., between two and four oxygen supplies, typically two oxygen supplies.

Typically, each photosynthetic oxygen supply comprises at least one light source and at least one, e.g., a pair, of layers of a photosynthetic source, e.g., algae. Typically, for applications in which a pair of layers of algae is used, the light source is disposed between first and second layers of the pair of algae layers.

The plurality of photosynthetic oxygen supplies are disposed as layers with respect to the support. Typically, the layers of algae in each oxygen supply provide a large surface area in which a ratio of the surface area of the algae to the surface area of the functional cells is greater than 1:1. An air gap is provided between each layer of photosynthetic oxygen supplies, and functions as a conduit for oxygen diffusion as well as a reservoir for storing excess oxygen produced by the oxygen generator. It is hypothesized by the inventors that an air gap is capable of storing at least four orders of magnitude more oxygen molecules relative to the oxygen-storage capacity of a liquid chamber.

The air gaps typically increase the diffusivity of the oxygen passing from the oxygen supply to the functional cells.

The photosynthetic oxygen supply typically comprises a light source and at least one layer of algae disposed upon an agarose matrix. Typically, the photosynthetic oxygen supply comprises a first and a second layer of algae, and the light source is disposed therebetween. It is to be noted that up to 12 layers of algae may be disposed on either side of the light source. In some applications, at least one of the layers of algae physically contacts the light source. Such configuration increases the surface area of the algae, allowing more light to contact the algae, and effectively facilitates increased oxygen production thereby. The photosynthetic oxygen supply is typically disposed between first and second layers of functional cells and is partitioned therefrom by respective first and second gas-permeable membranes. The first and second layers of functional cells are typically but not necessarily disposed at opposite ends of the housing. The gas within the housing is disposed at least in part in an air gap between the first layer of functional cells and the first layer of algae and in an air gap between the second layer of functional cells and the second layer of algae.

In some applications, a plurality of photosynthetic oxygen supplies are disposed within the housing. The oxygen supplies are typically arranged in layers between the first and second layers of functional cells. Each oxygen supply is separated from the next oxygen supply by an air gap. For applications in which the oxygen supply comprises fist and second layers of algae, each layer of algae is in contact with the gas in a respective air gap adjacent thereto.

Typically, the air gaps are configured to store and provide a buffer for the oxygen generated by the layers of algae. Typically, the algae produce oxygen at a generally constant rate while the functional cells consume oxygen at a variable rate. For applications in which the functional cells comprise islets of Langerhans, upon sensing a need for insulin, the functional cells consume oxygen at a higher rate during the production of insulin than at rest. Thus, the reservoirs provided by the air gap store the excess oxygen produced by the oxygen generator in order to meet the variable demand of the functional cells. Alternatively or additionally, the air gaps permit diffusion of the oxygen from the oxygen supplies to the functional cells. In such applications, the housing typically comprises a channel in communication with the air gaps which is configured to collect oxygen from the air gaps and to channel the oxygen toward the gas permeable membrane where it is ultimately diffused to the functional cells.

Typically, the oxygen generator is configured to supply oxygen to the functional cells and to sustain the functional cells for a period preceding vascularization around the housing, e.g., at least several weeks or months. During the period in which the oxygen generator supplies oxygen to the cells, the cells typically secrete factors that induce vascularization in fibrotic tissue surrounding the housing. Once the fibrotic tissue has become vascularized, the transplanted cells generally survive due to oxygen transfer from the newly-vascularized tissue, even in the absence of oxygen produced by the oxygen generator.

The use of a photosynthesizing system such as photosynthesizing algae and a light source is described in PCT Publication WO 01/50983 to Vardi et al., or US Patent Application Publication 2005/0136092 to Rotem.

In some applications, the support comprises a scaffold that houses the algae upon an agarose matrix.

In some applications, each layer of algae is disposed within a gas-permeable membrane and the membrane-enclosed algae are disposed adjacently to the light source.

For some applications, the membrane is coupled to the support in a manner in which an air gap is disposed between the layer of algae and the light source. In such applications, a first air gap is provided adjacent to the light-remote surface of the algae (i.e., between the surface of the algae disposed remotely with respect to the light source and the light-remote surface of a neighboring layer of algae), and a second air gap is provided adjacent to the light-adjacent surface of the algae (i.e., between light source and the surface of the algae disposed adjacent to the light source). The first and second air gaps allow the oxygen to diffuse into the housing through both surfaces of the layer (i.e., the light-remote surface and the light-adjacent surfaces of the layer), thereby increasing the diffusion rate of the oxygen from the algae layer. The algae produce most of the oxygen in the vicinity of the layer that is closest to the light source (i.e., the light- adjacent surface). The oxygen from the vicinity is allowed to diffuse into the air gap via the light-adjacent surface without having to diffuse through the .algae layer first before exiting toward the air gap via the light-remote surface.

For applications in which at least two photosynthetic oxygen supplies are disposed within the support, the layers of algae in each oxygen supply are relatively thin and have a width of between 150 um and 500 um, typically, about 200 um. The width of the algae layer allows for: (a) light to pass through the algae layer from the first oxygen supply, across an air gap and to the algae layer of the neighboring second oxygen supply, and (b) enhanced diffusion of the oxygen from the algae disposed adjacent to the light source, through the algae layer, and finally to the air gaps. The width of the algae layers reduces the time needed for oxygen transport through the algae layers, thereby facilitating increased diffusion of the oxygen through the layer and toward the air gaps. This increased diffusion enables the device to more rapidly supply sufficient oxygen to meet the oxygen consumption rate of the functional cells. Additionally, this diffusion of oxygen away from the algae disposed adjacently to the light source helps reduce the threat of oxygen toxicity for the algae disposed adjacently to the light source.

For applications in which the functional cells are islets of Langerhans, the oxygen supply typically supplies oxygen to the islets at a rate of between about 4 and 40 micromoles/hour per 100,000 transplanted islets, or as otherwise appropriate based on the type and number of functional cells and/or the body weight of the subject.

There is therefore provided, in accordance with some applications of the present invention, apparatus, including:
a housing, configured for implantation in a body of a subject;
functional cells, coupled to the housing;
a source of oxygen configured to supply oxygen to the functional cells; and
an oxygen delivery interface coupled to the housing, configured to receive oxygen from the source of oxygen, and to facilitate passage of the oxygen to the functional cells, while the housing is disposed within the body of the subject.

For some applications, the functional cells include cells disposed in pancreatic islets.

For some applications, the source of oxygen includes a plurality of gases.

For some applications, the source of oxygen includes oxygen carriers preloaded with oxygen.

For some applications, the oxygen delivery interface is reversibly couplable to the source of oxygen.

For some applications, the housing is configured to provide oxygen-containing gas in a volume sufficient to sustain the functional cells for a period of between 12 hours and 2 weeks.

For some applications, the housing is shaped to provide a plurality of projections which project into tissue of the subject, the projections being configured to absorb oxygen from vasculature of the subject.

For some applications, the functional cells are disposed in at least one layer of hydrogel configured to immunoisolate the cells from the body of the subject.

For some applications, the housing is shaped to provide an oxygen reservoir layer, and the functional cells are disposed in at least first and second layers of hydrogel, the first and second layers being disposed on either side of the oxygen reservoir layer.

For some applications, the oxygen reservoir layer has a longest dimension that is longer than a longest dimension of either of the first and second layers of the functional cells, and the oxygen reservoir layer provides surface area for absorbing oxygen from surrounding vasculature of the subject.

For some applications, the oxygen reservoir layer is shaped to provide a series of channels which facilitate directed transport of fluids within the oxygen reservoir layer.

For some applications, the oxygen reservoir layer includes at least one valve configured to facilitate directed transport of fluids within the oxygen reservoir layer.

For some applications, the housing is configured to be implanted in a vicinity of a ribcage of the subject, and the housing is configured to circulate the oxygen in the reservoir layer in response to movements of the housing responsively to movements of the ribcage of the subject.

For some applications, the oxygen reservoir layer includes a hydrogel shaped to define a channel configured to facilitate directed transport of oxygen within the reservoir layer.

For some applications, the oxygen reservoir layer includes the oxygen delivery interface and is couplable to and receives oxygen from the source of oxygen.

For some applications, the oxygen delivery interface includes an interface between the reservoir layer and one of the first and second layers of cells.

For some applications, the oxygen reservoir layer includes a gas.

For some applications, the oxygen reservoir layer includes oxygen carriers.

For some applications:
during a first time, the interface is configured to facilitate:
   coupling of the source of oxygen to the housing,
   supplying of oxygen from the source of oxygen to the cells coupled to the housing, and
   decoupling of the source of oxygen from the interface following the supplying of oxygen, and
at a second time, the interface is configured to facilitate:
   coupling of the source of oxygen to the housing,
   supplying of oxygen from the source of oxygen to the cells coupled to the housing, and
   decoupling of the source of oxygen from the interface following the supplying of oxygen.

For some applications:
the interface includes a penetrable surface,
the apparatus further includes a needle configured for transcutaneously penetrating the surface with the needle,
the housing is indirectly couplable to the source of oxygen via the needle, and
the needle facilitates supplying of oxygen to the cells from the source of oxygen.

For some applications, the housing is shaped to define an upper surface, and the penetrable surface includes the upper surface of the housing.

For some applications, the apparatus includes at least one oxygen-delivery port having an upper surface thereof and a tube coupled at a first end thereof to the port and at a second end thereof to the housing, wherein the upper surface of the port includes the penetrable surface that is penetrable by the needle.

For some applications, the port is coupled to an upper surface of the housing.

For some applications, the port is disposed remotely from the housing.

For some applications:
the interface includes at least one fluid inlet tube in fluid communication with the housing,
the housing is indirectly couplable to the source of oxygen via the fluid inlet tube, and
the tube facilitates supplying of oxygen to the cells from the source of oxygen.

For some applications, the fluid inlet tube is configured to be disposed transcutaneously in the body of the subject.

For some applications, the housing is shaped to define an oxygen reservoir to store oxygen provided to the housing from the source of oxygen.

For some applications, the oxygen reservoir is configured to store fluid at a pressure of at least 1 atm.

For some applications, a volume of the reservoir is between 100 ml and 300 ml.

For some applications, the oxygen reservoir includes a gas.

For some applications, the oxygen reservoir includes oxygen carriers configured to absorb excess oxygen disposed in the oxygen reservoir.

For some applications, the apparatus includes a gas-permeable membrane disposed between the reservoir and the functional cells.

For some applications:
the oxygen delivery interface includes at least one fluid inlet tube in fluid communication with the housing, and
a first end of the tube is reversibly couplable to the source of oxygen, in a manner that allows the source of oxygen to supply oxygen to the functional cells via the tube.

For some applications, the fluid inlet tube is configured to be disposed transcutaneously in the body of the subject.

For some applications, the apparatus includes a fluid outlet tube in communication with the housing, and the fluid outlet tube is configured to facilitate passage of fluid from within the housing to a site external to the housing.

For some applications, the fluid outlet tube is configured to be disposed transcutaneously in the body of the subject.

For some applications, the fluid outlet tube is configured to facilitate passive passage of the fluid from within the housing, in conjunction with the supplying of oxygen to the housing by the fluid inlet tube.

For some applications, the apparatus further includes a source of suction configured to facilitate active drawing of fluid from within the housing.

For some applications, the oxygen delivery interface includes a penetrable surface.

For some applications, the apparatus includes a needle having at least one chamber, the needle is couplable to the source of oxygen and is configured for transcutaneous penetration of the penetrable surface of the housing.

For some applications, the at least one chamber is configured to facilitate delivery of oxygen from the source of oxygen to the functional cells coupled to the housing.

For some applications:
the at least one chamber includes a first chamber and a second chamber,
the first chamber is configured to facilitate delivery of oxygen from the source of oxygen to the functional cells coupled to the housing, and
the second chamber is configured to facilitate passage of fluid from within the housing to outside the body of the subject.

For some applications, the second chamber is configured to facilitate passive passage of the fluid from within the housing, in conjunction with the supplying of oxygen to the housing by the first chamber.

There is additionally provided, in accordance with some applications of the present invention, a method for use with apparatus including an implantable housing including functional cells and an oxygen delivery interface, the method including:
at a first time:
   coupling a source of oxygen to the interface;
   supplying oxygen from the source of oxygen to the cells; and
   decoupling the source of oxygen from the interface; and
at a second time:
   coupling the source of oxygen to the interface; supplying oxygen from the source of oxygen to the cells; and
   decoupling the source of oxygen from the interface.

For some applications, the functional cells include cells disposed in pancreatic islets of Langerhans, and supplying oxygen from the source of oxygen to the cells includes supplying oxygen from the source of oxygen to the islets.

For some applications, the method includes facilitating passive passage of fluid from the housing in conjunction with the supplying of oxygen to the housing during the first and second times.

For some applications, the method includes actively drawing fluid from the housing in conjunction with the supplying of oxygen to the housing during the first and second times.

For some applications, the method includes regulating a rate of oxygen transport from the reservoir to the functional cells.

For some applications, regulating the rate of oxygen transport includes providing oxygen carriers in the reservoir which absorb excess oxygen in the reservoir and release the oxygen in a low oxygen environment.

There is further provided, in accordance with some applications of the present invention, apparatus, including:
a housing, configured for implantation in a body of a subject, the housing:
   shaped to define an oxygen reservoir, and
   including a flexible upper surface thereof;
functional cells, coupled to the hosing; and
a tube coupled to the housing, the tube having a first end thereof that is in fluid communication with the reservoir and a second end thereof configured to be in fluid communication with a trachea of the subject, the tube being configured to facilitate oxygen transport to the functional cells in response to a pushing force applied to the upper surface of the housing.

For some applications, the housing is configured to be disposed remotely from the trachea.

For some applications, the upper surface is configured to facilitate pumping of gas from the trachea into the housing.

For some applications, the upper surface, in response to the pushing force applied thereto, is configured to force air out of the reservoir and through the tube toward the trachea.

For some applications, following the pushing force applied to the upper surface, the upper surface is configured to reduce a pressure in the reservoir and draw air into the reservoir from the trachea via the tube.

For some applications, the functional cells are disposed in at least one layer of hydrogel configured to immunoisolate the cells from the body of the subject.

For some applications, the method further includes provided an oxygen reservoir layer, and the functional cells are disposed in first and second layers of hydrogel, the first and second layers being disposed on either side of the oxygen reservoir layer.

For some applications, the reservoir layer is configured to absorb oxygen from surrounding vasculature of the subject.

For some applications, the oxygen reservoir layer is in communication with the first end of the tube and receives oxygen from the trachea via the tube.

There is also provided, in accordance with some applications of the present invention a method, including:
subcutaneously implanting in a body of a subject, a housing shaped to define an oxygen reservoir and shaped to provide a flexible upper surface that is in contact with subcutaneous tissue of the subject;
implanting a first portion of a tube in a trachea of the subject, the tube having a second end in communication with the oxygen reservoir of the housing; and
facilitating transport of oxygen from the trachea into the reservoir of the housing and toward the cells by applying a pushing force to the upper surface of the housing.

For some applications, applying the pushing force includes forcing the air out of the reservoir and into the trachea of the subject.

There is yet further provided, in accordance with some applications of the present invention, apparatus, including:
a housing, configured for insertion into a body of a subject;
one or more photosynthetic oxygen supplies, disposed within the housing;
at least one layer of functional cells, disposed within the housing, and configured to receive oxygen from the one or more oxygen supplies; and
a gas disposed to permit passage therethrough of the oxygen from the one or more oxygen supplies to the cells, the gas disposed at least in part between the layer of functional cells and at least one of the oxygen supplies.

For some applications, the functional cells include pancreatic islet cells.

For some applications, the gas includes oxygen and carbon dioxide.

For some applications, the housing includes a semi-permeable membrane having associated therewith a molecular weight cutoff, the membrane being disposed with respect to the functional cells so as to protect the functional cells from components of a body fluid of the subject having molecular weights higher than the cutoff.

For some applications, the membrane includes a gas-permeable membrane.

For some applications, each photosynthetic oxygen supply includes:
a light source; and
at least one first layer of algae, positioned to receive light from the light source.

For some applications, the apparatus includes a membrane disposed between the oxygen supply and the functional cells, the membrane having associated therewith a molecular weight cutoff configured to restrict passage of algae therethrough.

For some applications, the light source has an input electrical power between 5 mW/(cm^2 of algae) and 50 mW/(cm^2 of algae).

For some applications, an energy of illumination applied to the algae by the light source is at a rate of between 0.2 mW/(cm^2 of algae) and 2.0 mW/(cm^2 of algae).

For some applications, the first layer of algae has a width of between 100 um and 2000 um.

For some applications, the first layer of algae has a width of between 100 um and 300 um.

For some applications, the at least one layer of algae is in contact with the gas.

For some applications, the gas is disposed in part between the layer of algae and the light source.

For some applications, the at least one layer of algae includes a first and a second layer of algae, and the light source is disposed between the first and second layers.

For some applications, both the first and second layers of algae are in contact with the gas.

For some applications, the one or more layers of functional cells include a first and a second layer of functional cells, and the first and second layers of functional cells are in communication with the first and second layers of algae.

For some applications, the first layer of functional cells is spaced apart from the first layer of algae by a distance of between 1000 um and 6000 um.

For some applications, the first layer of functional cells is spaced apart from the first layer of algae by a distance of between 100 um and 500 um.

For some applications, the gas is disposed at least in part between the first layer of algae and the first layer of functional cells, and between the second layer of algae and the second layer of functional cells.

For some applications, the first layer of functional cells is spaced apart from the first layer of algae by a distance of between 1 um and 1000 um.

For some applications, the light source includes a first light source configured to be in optical communication with at least a first surface of the first layer of algae, and the apparatus further includes a second light source configured to be in optical communication with at least the first surface of the first layer of algae, the first layer of algae being disposed between the first and second light sources.

For some applications:
the apparatus includes a second layer of algae, the second layer of algae being in optical communication with the second light source,
the gas is disposed at least in part between the first and second layers of algae, and
light from the second light source travels through the second layer of algae and toward the first surface of the first layer of algae.

For some applications:
the one or more photosynthetic oxygen supplies includes first and second oxygen supplies,
the first oxygen supply includes the first layer of algae and the first light source,
the second oxygen supply includes the second layer of algae and the second light source,
light from the first light source is configured to provide light for the first layer of algae and for at least a portion of the second layer of algae, and
light from the second light source is configured to provide light for the second layer of algae and at least a portion of the first layer of algae.

For some applications, the second layer of algae has a width of between 100 um and 2000 um.

For some applications, the second layer of algae has a width of between 100 um and 3 00 um.

For some applications, the one or more photosynthetic oxygen supplies include a plurality of oxygen supplies.

For some applications, each of the plurality of oxygen supplies is spaced apart from an adjacent one of the oxygen supplies by an area, a width thereof being between 100 um and 300 um.

For some applications, the apparatus includes a channel in communication with the area, the channel configured to collect a portion of the oxygen from the area and to transport the oxygen to the functional cells.

For some applications, the gas is disposed at least in part between each photosynthetic oxygen supply.

For some applications, each photosynthetic oxygen supply includes:
a light source; and
at least one layer of algae, positioned to receive light from the light source.

For some applications, the at least one layer of algae is in contact with the gas.

For some applications, the at least one layer of algae includes a first and a second layer of algae, and the light source is disposed between the first and second layers of algae.

For some applications, both the first and second layers of algae are in contact with the gas.

There is still further provided, in accordance with some applications of the present invention, apparatus, including:
a housing having at least one outlet;
one or more photosynthetic oxygen supplies disposed within the housing and in communication with the outlet; and
a gas disposed to permit passage therethrough of oxygen generated from the one or more oxygen supplies toward the outlet, the gas disposed at least in part between the outlet and at least one of the oxygen supplies.

For some applications, each photosynthetic oxygen supply includes:
a light source; and
at least one first layer of algae, positioned to receive light from the light source.

For some applications, the light source has an input electrical power between 5 mW/(cm^2 of algae) and 50 mW/(cm^2 of algae).

For some applications, an energy of illumination applied to the layer of algae is at a rate of between 0.2 mW/(cm^2 of algae) and 2.0 mW/(cm^2 of algae).

For some applications, the first layer of algae has a width of between 100 um and 2000 um.

For some applications, the first layer of algae has a width of between 100 um and 300 um.

For some applications, the light source includes a first light source configured to be in optical communication with at least a first surface of the first layer of algae, and the apparatus further includes a second light source configured to be in optical communication with at least the first surface of the first layer of algae, the first layer of algae being disposed between the first and second light sources.

For some applications:
the apparatus includes a second layer of algae, the second layer of algae being in optical communication with the second light source,
the gas is disposed at least in part between the first and second layers of algae, and
light from the second light source travels through the second layer of algae and toward the first surface of the first layer of algae.

For some applications:
the one or more photosynthetic oxygen supplies includes first and second oxygen supplies,
the first oxygen supply includes the first layer of algae and the first light source,
the second oxygen supply includes the second layer of algae and the second light source,
light from the first light source is configured to provide light for the first layer of algae and for at least a portion of the second layer of algae, and
light from the second light source is configured to provide light for the second layer of algae and at least a portion of the first layer of algae.

For some applications, the second layer of algae has a width of between 100 um and 2000 um.

For some applications, the second layer of algae has a width of between 100 um and 300 um.

For some applications, the at least one layer of algae is in contact with the gas.

For some applications, the gas is disposed in part between the layer of algae and the light source.

For some applications, the at least one layer of algae includes a first and a second layer of algae, and the light source is disposed between the first and second layers.

For some applications, both the first and second layers of algae are in contact with the gas.

For some applications, both the first and second layers of algae are in contact with the light source.

For some applications, the one or more photosynthetic oxygen supplies include a plurality of oxygen supplies.

For some applications, the gas is disposed at least in part between each photosynthetic oxygen supply.

For some applications, the housing is configured for insertion into a body of a subject, and the gas is configured to permit passage therethrough of the oxygen from the photosynthetic oxygen supply toward the outlet and subsequently to a vicinity in the body of the subject that is external to the housing.

For some applications, the housing includes a semi-permeable membrane having associated therewith a molecular weight cutoff, the membrane being disposed with respect to the housing so as to protect the photosynthetic oxygen supply from components of a body fluid of the subject having molecular weights higher than the cutoff.

For some applications, the membrane includes a gas-permeable membrane.

There is additionally provided, in accordance with some applications of the present invention, apparatus, including:
a housing;
at least a first layer of a photosynthetic source disposed within the housing, the layer having at least a first surface;
at least a first light source in optical communication with the first surface of the first layer of the photosynthetic source;
at least a second light source in optical communication with the first surface of the first layer of the photosynthetic source; and
a gas disposed to permit passage therethrough of oxygen from the first layer of the photosynthetic source.

For some applications, the first surface of the photosynthetic source is in contact with the gas.

For some applications, the photosynthetic source is in contact with the first light source.

For some applications, the first and second light sources each have an input electrical power between 5 mW/(cm^2 of algae) and 50 mW/(cm^2 of algae).

For some applications, an energy of illumination applied to the algae by the first and second light sources is at a rate of between 0.2 mW/(cm^2 of algae) and 2.0 mW/(cm^2 of algae).

For some applications, the layer of the photosynthetic source has a width of between 100 um and 2000 um.

For some applications, the layer of the photosynthetic source has a width of between 100 um and 300 um.

For some applications:
the at least a first layer of the photosynthetic source includes first and second layers of photosynthetic sources, the second layer of the photosynthetic source being in optical communication with the second light source,
the gas is disposed at least in part between the first and second layers of the photosynthetic sources, and
light from the second light source travels through the second layer and toward the first surface of the first layer.

For some applications, the second layer has a width of between 100 um and 2000 um.

For some applications, the second layer has a width of between 100 um and 300 um.

For some applications, the housing includes at least one layer of functional cells configured to receive oxygen from the photosynthetic source, and the gas is disposed at least in part between the layer of cells and the photosynthetic source and is configured to permit passage therethrough of the oxygen from the photosynthetic source to the cells.

For some applications, the functional cells include pancreatic islet cells.

For some applications, the photosynthetic source includes algae, and the apparatus further includes a membrane disposed between the photosynthetic source and the functional cells, the membrane having associated therewith a molecular weight cutoff configured to restrict passage of algae therethrough.

For some applications, the membrane includes a gas-permeable membrane.

For some applications, the housing is configured for insertion into a body of a subject, and the gas is configured to permit passage therethrough of the oxygen from the photosynthetic source toward a vicinity in the body of the subject that is external to the housing.

For some applications, the housing includes a semi-permeable membrane having associated therewith a molecular weight cutoff, the membrane being disposed with respect to housing so as to protect the photosynthetic source from components of a body fluid of the subject having molecular weights higher than the cutoff.

For some applications, the membrane includes a gas-permeable membrane.

The present invention will be more fully understood from the following detailed description of applications thereof, taken together with the drawings, in which:

### BRIEF DESCRIPTION OF THE DRAWINGS

Figs. 1A-B are schematic illustrations of a housing comprising a penetrable surface and housing functional cells;
Fig. 2 is a schematic illustration of a housing coupled to a plurality of fluid-delivery ports;
Fig. 3 is a schematic illustration of the housing coupled to a plurality of fluid delivery ports, in accordance with some other applications of the present invention;
Figs. 4A-B are schematic illustrations of an oxygen reservoir layer surrounded by two layers of functional cells;
Figs. 5A-C are schematic illustrations of a housing indirectly coupled to a trachea of a subject;
Figs. 6 and 7 are schematic illustrations of a housing coupled to a fluid inlet tube and a fluid outlet tube and housing functional cells;
Fig. 8 is a schematic illustration of an oxygen generator;
Fig. 9 is a schematic illustration of a housing comprising the oxygen generator of Fig. 1 and layers of functional cells;
Figs. 10-12 are graphical representations of calculated, simulated parameters of the oxygen generator;
Fig. 13 is a schematic illustration of a flow of oxygen between the oxygen generator and the functional cells; and
Fig. 14 is a schematic illustration of the housing comprising the oxygen generator.

### DETAILED DESCRIPTION OF THE DRAWINGS

Reference is now made to Figs. IA-B, which are schematic illustrations of a system 20 comprising a subcutaneously-implantable housing 24 containing a layer 32 of functional cells, e.g., disposed in transplanted pancreatic islets of Langerhans. Housing 24 comprises a scaffold 25, e.g., silicone or metal, which separates layer 32 of functional cells from a surface 28 comprising a penetrable material, e.g., rubber, silicone, orplastic. Typically an upper surface 31, i.e., the surface of housing 24 that is in contact with subcutaneous tissue of subject 22, comprises penetrable surface 28. Surface 28 serves as an oxygen delivery interface 27, which by being punctured facilitates access to housing 24 by a disposable needle 50. A source of oxygen (not shown), e.g., a vessel such as a syringe, comprising a source of fluid which comprises oxygen, is coupled to needle 50 and supplies via needle 50 fluid containing oxygen to the functional cells (e.g., in islets) that are disposed within housing 24. In some applications, the source of oxygen comprises air. Alternatively, the source of oxygen comprises pure oxygen.

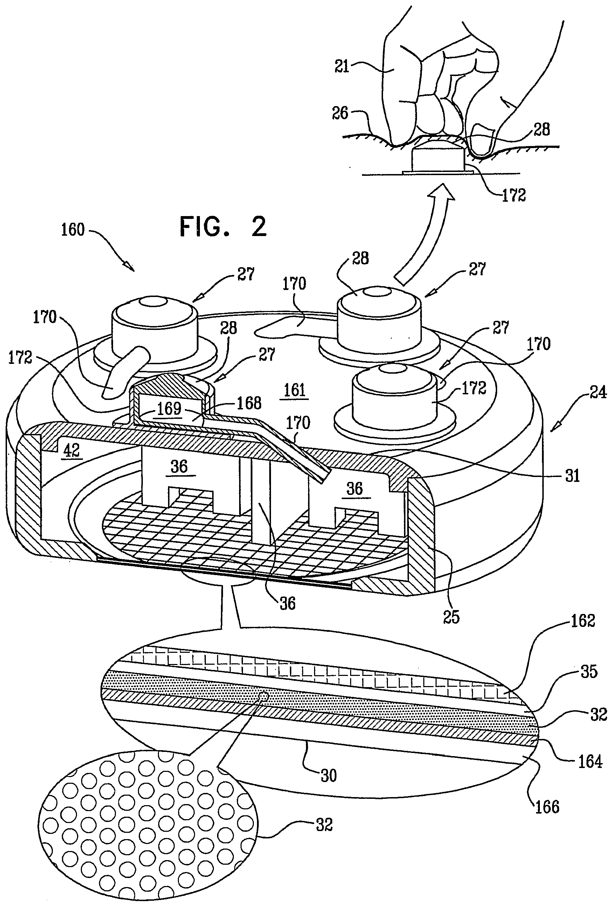

Typically, layer 32 comprises between 40,000 and 400,000 islets, e.g., typically, 400,000 islets, which are evenly distributed in *a* single layer 32. As appropriate for a given application, the number of islets may also be outside of this range. In some applications, the islets are arranged in a manner in which every second row of islets in layer 32 is offset with respect to its neighboring row of islets (configuration shown in Fig. 2). Oxygen is typically supplied to the islets in layer 32 in a volume and concentration in which the partial pressure of oxygen in each islet is between 8 and 40 µM, or between 40 and 200 µM.

In some applications, the source of oxygen comprises a plurality of gases including oxygen. The gases are disposed in the container at a pressure of 1 atm or higher. Typically, the source of oxygen comprises around 5% carbon dioxide in order to maintain a balance of concentrations of carbon dioxide inside the housing and outside the housing. For some applications, the source of oxygen comprises a fluid comprising oxygen carriers (e.g., hemoglobin-based oxygen carriers such as chemically-modified hemoglobin, or "microbubbles" which comprise fluorocarbons such as dodecafluoropentane or perfluorodecalin) that are loaded with oxygen prior to the injection of the carriers into housing 24. The carriers facilitate the transport into housing 24 of a larger volume of compressed oxygen.

Typically, the functional cells of layer 32 are disposed in a layer of liquid or gel, such as alginate, agarose, or polyethylene glycol (PEG) and/or dispersed in a three-dimensional biodegradable or non-biodegradable fibrillar matrix. In some applications, layer 32 comprises an alginate slab which houses the functional cells and functions to imrnunoisolate the cells. This application may be practiced in combination with techniques described in US Provisional Patent Application 61/192,412 to Barkai et al..

Layer 32 has an interface 30 in contact with the body of a subject 22. Typically, insulin and/or other by-products of the functional cells are released to the body through interface 30. In some applications, interface 30 comprises a surface of the alginate slab. In some applications, interface 30 comprises a selectively-permeable membrane which immunoisolates the transplanted cells as well as facilitates transport of (a) molecules, e.g., insulin, from the cells to the body of subject 22, and (b) molecules, e.g., glucose, from the body of subject 22 to the cells in housing 24.

Scaffold 25 defines a space comprising an oxygen reservoir 42, typically having a volume of between 100 ml and 300 ml, e.g., between 150 ml and 200 ml. Oxygen reservoir 42 comprises foam, e.g., an open-cell silicone foam, or simply an air gap which functions as a gas reservoir within the device. Reservoir 42 functions as a conduit for oxygen diffusion to the functional cells, as well as a reservoir for storing excess oxygen that is supplied to the housing by the source of oxygen. Techniques described herein with respect to reservoir 42 may be practiced in combination with techniques described with respect to an air gap in PCT Patent Application PCT/1L08/001204 to Stern et al..

In some applications, the housing comprises the oxygen carriers. In such applications, the oxygen carriers function to store, or carry, oxygen when in excess, and release the oxygen upon a need therefor.

Housing 24 is shown as being disc-shaped by way of illustration and not limitation. For example, housing 24 may be rectangular or any other suitable shape suitable for implantation under skin 26 of subject 22. In some applications, housing 24 is shaped to provide a plurality of projections which project radially from housing 24 and toward a vicinity of the body of subject 22 which includes vasculature. In such applications, the projections function as oxygen delivery interface 27 by providing increased surface area of housing 24 for facilitating transport of oxygen from surrounding vasculature toward housing 24. For some applications, the projections of housing 24 contain the oxygen carriers, which store excess oxygen that has been absorbed into housing 24 by the projections.

At some time following implantation of housing 24 in the body of subject 22, housing 24 is primed with a suitable amount of oxygen-containing fluid. (The housing may have previously been filled with oxygen-containing fluid, as well.) Typically, the subject 22 transcutaneously punctures surface 28 of housing 24 using needle 50, and advances a distal tip of needle 50 through reservoir 42 and toward layer 32 of functional cells. Contact between layer 32 of cells and the distal tip of needle 50 is prevented by a substantially rigid separating layer 34 that is disposed above and protects layer 32 of functional cells. A plurality of vertical supports 36 are disposed between separating layer 34 and surface 28 of housing 24.

For some applications, separating layer 34 is shaped to define a plurality of channels 38 and 40 which facilitate bidirectional transport of oxygen (a) from reservoir 42 toward layer 32 of cells, and (b) carbon dioxide from layer 32 of cells toward reservoir 42. Alternatively, oxygen travels from reservoir 42 to layer 32 by other routes.

In some applications, housing 24 comprises a gas-permeable membrane 35, e.g., a Millipore membrane and/or a membrane comprising silicone, that is disposed between layer 32 of functional cells and separating layer 34. Gas-permeable membrane 35 facilitates the transport of gases to and from layer 32 of functional cells. Typically, membrane 35 has a width and a pore size which regulates a rate of transport of gases to and from layer 32.

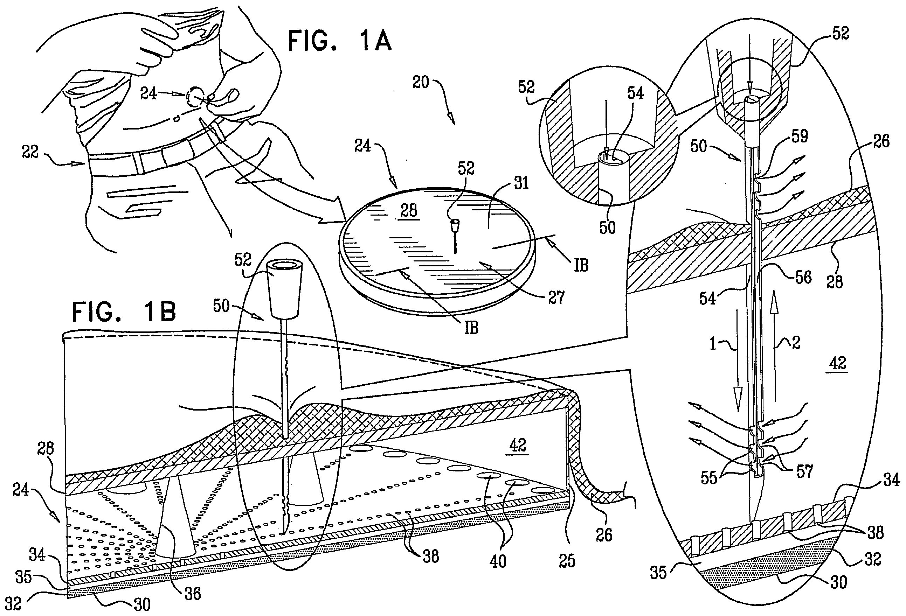

An enlarged image of Fig. 1B shows a cross-sectional illustration of needle 50. Needle 50 comprises a double-chambered needle comprising a fluid inlet chamber 54 and a fluid outlet chamber 56. A proximal end of needle 50 is coupled to a luer connector 52 which facilitates the coupling of a vessel (e.g., a syringe) comprising oxygen-containing fluid. Luer connector 52 is in fluid communication with a proximal end of fluid inlet chamber 54. The fluid is actively expelled from the syringe through chamber 54 and exits chamber 54 via one or more perforations 55 at the distal end of needle 50, in order to enter reservoir 42 of housing 24.

When fluid comprising a high oxygen content is actively injected into reservoir 42 (i.e., in a direction as indicated by arrow 1), fluid disposed within reservoir 42 having a low oxygen content passively exits reservoir 42 through one or more perforations 57 in fluid outlet chamber 56. The fluid having a low oxygen content exits needle 50 by traveling in a direction as indicated by arrow 2, and through one or more perforations 59 at a proximal portion of chamber 56 that is disposed outside a surface of skin 26 of subject 22.

It is to be noted that needle 50 comprises three of each perforations 55, 57, and 59 by way of illustration and not limitation, and that needle 50 may be shaped to define any suitable number of perforations 55, 57, and 59.

It is to be noted that the plurality of perforations 55 of chamber 54 are disposed with respect to the plurality of perforations 57 of chamber 56, at substantially the same cross-sectional plane of needle 50 by way of illustration and not limitation. For example, perforations 55 may be disposed at a more proximal site of the intracorporeal portion of needle 50 while perforations 57 are disposed at a more distal site of the intracorporeal portion of needle 50.

Typically, fluid having a high oxygen content is injected into reservoir 42 in excess. In some applications, the excess oxygen is in the form of free gas. Alternatively, the excess oxygen is loaded onto oxygen carriers, as described hereinabove. Providing the oxygen in excess is done in order to ensure that sufficient oxygen-containing fluid remains within reservoir 42 following the passive transport of fluid through fluid outlet chamber 56 (which occurs in conjunction with the injecting).

Typically, oxygen is supplied to housing 24 in a volume and concentration in accordance with the size of housing 24 and with the amount of functional cells disposed therein. Additionally, the amount of oxygen delivered to housing 24 depends on the composition of the fluid injected into housing 24. That is, a given volume of fluid comprising preloaded oxygen carriers will sustain the functional cells in housing 24 for a longer period than will the same volume of fluid comprising free oxygen. In general, oxygen delivery interface 27 facilitates the provision, on a recurring basis, of oxygen to the functional cells in a volume and concentration sufficient to meet the oxygen consumption rate of the functional cells over a given period of time, e.g., between 12 hours and 2 weeks.

The following table depicts, by way of illustration and not limitation, the projected parameters relating to the source of oxygen with respect to the size of housing 24 comprising 400,000 islets in various distributions thereof within housing 24:

| Cell layer # | CTC (um) | ID | Diam. | K Alginate (mm^2/s) | Alginate slab thickness (um) | P Min (uM) | W (mm) |
|---|---|---|---|---|---|---|---|
| 1 | 155 | 4808 | 103 | 1.5E-03 | 250 | 180 | 91 |
| 1 | 165 | 4237 | 110 | 1.5E-03 | 250 | 161 | 52 |
| 1 | 175 | 3769 | 116 | 1.5E-03 | 250 | 145 | 36 |
| 1 | 175 | 3769 | 116 | 2.5E-03 | 250 | 108 | 23 |
| 1 | 175 | 3769 | 116 | 1.5E-03 | 200 | 125 | 28 |
| 1 | 185 | 3769 | 116 | 1.5E-03 | 200 | 90 | 20 |
| 1 | 185 | 3371 | 123 | 1.5E-03 | 250 | 133 | 27 |
| 2 | 265 | 3290 | 124 | 1.5E-03 | 350 | 176 | 56 |

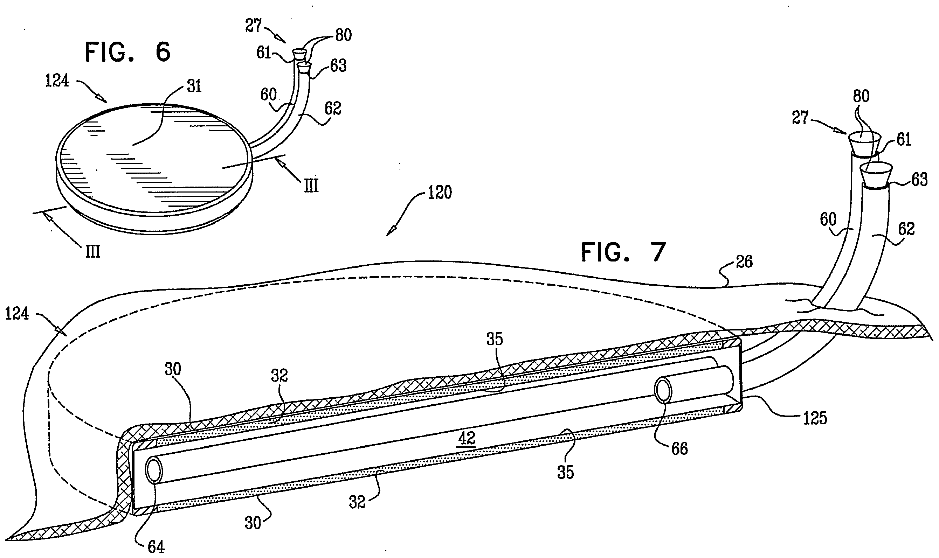

where:
Cell layer # is the number of cell layers (either one (as in Fig. 1B) or two (as in Fig. 7));
CTC is the center to center distance between the islets, i.e., the distance from the center of one islet to the center of a neighboring islet;
ID is islet density (islets/cm2);
Diam. is the diameter of each alginate slab (mm);
K Alginate is the permeation coefficient of oxygen in the alginate of the alginate slab (mm^2/s);
P min is the minimum partial pressure of oxygen in reservoir 42, such that the partial pressure of oxygen within each islet is between 8 and 200 uM, which is sufficient for sustaining functional islets. Typically, the oxygen in reservoir 42 may be depleted to a minimum of about 90 uM and subsequently restored to about 215 uM (corresponding to about 21% oxygen). It is to be noted that for some applications, the partial pressure may be restored to a value greater than 215 uM, e.g., reservoir 42 may contain 21%-50% or 50%-100% oxygen; and
W is the thickness/width of reservoir 42.

Typically, housing 24 comprises a single layer 32 of functional cells disposed in a single, thin alginate slab having a high permeation coefficient. Typically, layer 32 of functional cells comprises 300,000-500,000 islets of Langerhans (e.g., around 400,000) having a density of between 3200 and 5000 islets/cm^2, e.g., 3700-4000 islets/cm^2.

In general, housing 24 has a diameter of between 100 mm and 150 mm, e.g., 125 mm, and a width of between 20 mm and 100 mm, e.g., 25 mm. Housing 24 is typically primed with air comprising 21% oxygen. Oxygen is typically supplied to housing 24 in a concentration between 30 and 500 uM, e.g., 57-215 uM. Housing 24 is configured to contain gases at a pressure of 1 atm or greater.

At regular intervals (e.g., at least once a week, typically, once a day), subject 22 reintroduces needle 50 within housing 24 via oxygen delivery interface 27. At such intervals, a source of oxygen (e.g., a syringe or pre-filled cartridge) is connected to needle 50 and supplies oxygen-containing fluid to the islets in housing 24.

It is to be noted that needle 50 is shown as comprising a double-chambered needle by way of illustration and not limitation. For example, needle 50 may comprise a single-chambered needle. In such applications, the source of oxygen may be coupled to a pump which facilitates cycling between (1) gradually actively injecting a portion of fluid having a high oxygen content from the source of oxygen and into housing 24, and (2) gradually actively drawing (or passively allowing) fluid having a low oxygen content out of housing 24. For some applications, the user may perform the same actions as described herein for performance by the pump.

It is to be noted that penetrable surface 28 of housing 24 may define only a portion of the upper surface of housing 24. In such applications, the upper surface of housing 24 comprises a port region which comprises penetrable surface 28.

Fig. 2 shows a system 160 comprising subcutaneously-implantable housing 24 coupled to a plurality of fluid-injection ports 172, in accordance with some applications of the present invention. Upper surface 31 comprises a rigid, impenetrable surface 161 that is supported by a plurality of mechanical supports 36. Ports 172 are disposed upon upper surface 31 and comprise a rigid, impenetrable base 168 and walls which define a port chamber 169. An upper surface of each port comprises a penetrable surface 28, which functions as oxygen delivery interface 27. A respective tube 170 facilitates transfer of oxygen from ports 172 to reservoir 42 and toward layer 32 of islets. Each tube 170 provides: (a) a first end coupled to port 172 and in fluid communication with chamber 169, (b) a body portion which crosses upper surface 31 or the side of housing 24, and (c) a second end that is in fluid communication with reservoir 42 of housing 24.

A protective grid 162 is disposed at a lower portion of housing 24. Grid 162 provides a base for supports 36. For some applications, gas-permeable membrane 35 is disposed between grid 162 and layer 32 of islets, and facilitates the transport of gases between reservoir 42 and layer 32. A second grid 164 is disposed beneath layer 32 of islets and provides support therefor. A selectively-permeable membrane 166 is typically disposed at interface 30 of housing 24 with tissue of subject 22. Selectively-permeable membrane 166 comprises a Millipore membrane having a pore size of typically, about 0.5 um, immunoisolates the transplanted cells, and facilitates transport of (a) molecules, e.g., insulin, from the cells to the body of subject 22, and (b) molecules, e.g., glucose, from the body of subject 22 to the cells in housing 24.

Typically, following implantation of housing 24 in the body of subject 22, housing 24 is primed with a suitable amount of oxygen-containing fluid. (The housing may have previously been filled with oxygen-containing fluid, as well.) Typically, subject 22 feels for at least one port 172 with his or her hand 21. Upon determining the location of the port, subject 22 transcutaneously punctures skin 26 with a first needle and subsequently penetrates penetrable surface 28 of a first port 172. In such applications, the needle comprises a single-chambered needle. Typically, subject 22 then feels for a second port 172 and transcutaneously punctures surface 28 of the second port 172 with a second single-chambered needle. Impenetrable base 168 of each port 172 together with impenetrable surface 161 of housing 24 prevent passage of the needles into housing 24. The first needle is used to draw fluid from within housing 24 by lowering the pressure in chamber 169 of the first port 172. In response to reducing the pressure, fluid is drawn from reservoir 42, through tube 170, into chamber 169 of the first port 172, and finally through the first needle. In conjunction with drawing the fluid, the second needle is coupled to a source of oxygen, as described hereinabove, and facilitates the passage of oxygen-containing fluid into housing 24.

In some applications, oxygen-containing fluid is driven through the first needle, and oxygen-depleted fluid that had previously been in the housing is thus driven out of housing 24, to be replaced by the oxygen-containing fluid.

For some applications, a common structure holds both the first and second needles prior to and during their puncturing of the skin and surface 28 of each port.

In some applications, upper surface 31 of housing 24 is flexible and penetrable. In such applications, only the respective bases 168 of each port prevent passage of the needle into housing 24.

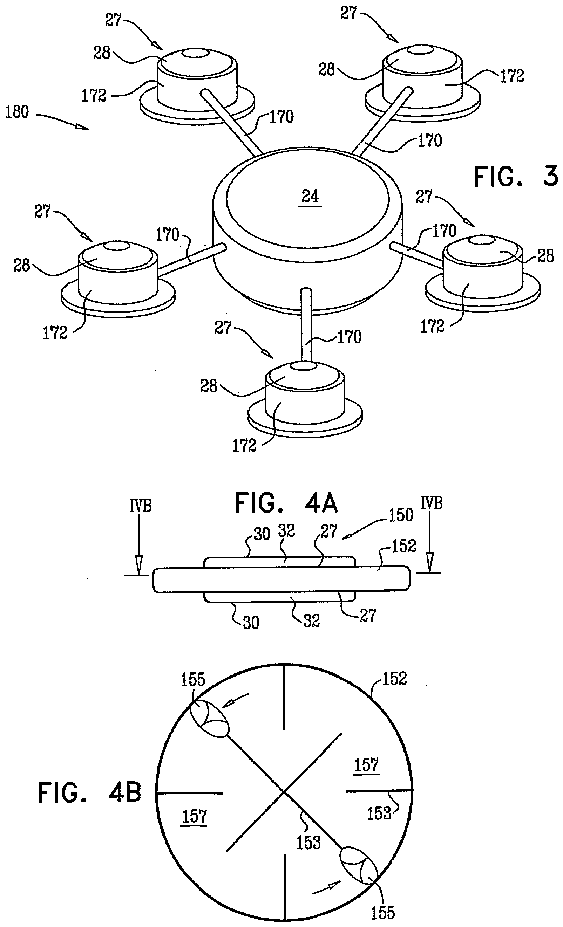

Reference is now made to Fig. 3, which is a schematic illustration of a system 180 similar to system 160 described hereinabove with reference to Fig. 2, with the exception that ports 172 are disposed remotely with respect to housing 24. The remote positioning of ports 172 with respect to housing 24 facilitates the delivery of fluid to housing 24 without substantially shifting the position of housing 24.

Reference is now made to Figs. 4A-B, which are schematic illustrations of a system 150 comprising two layers 32 of functional cells which surround an oxygen reservoir layer 152, in accordance with some applications of the present invention. Layers 32 of functional cells comprise islets that are typically evenly distributed with respect to layers 32 in a manner as described hereinabove. Reservoir layer 152 receives and stores oxygen-containing fluid (e.g., gas comprising molecules of oxygen, or a liquid comprising oxygen carriers preloaded with oxygen). In some applications, layer 152 comprises a hydrogel, e.g., alginate. In some applications, layer 152 comprises a gas reservoir.

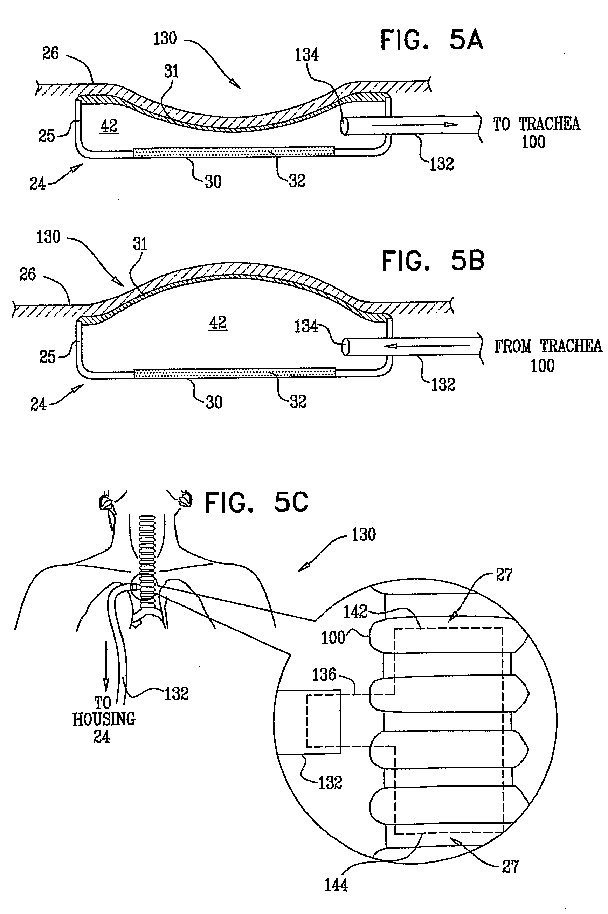

In some applications, reservoir layer 152 receives the oxygen-containing fluid from the source of oxygen via a needle (e.g., in a manner as described hereinabove with reference to Figs. 1A-B and 2-3), or via a fluid-inlet tube (e.g., in a manner described hereinbelow with reference to Figs. 5A-C and 6-7). It is to be noted that system 150 may be used independently or in combination with applications described in Figs. 1A-B, 2-3, 5A-C, and 6-7 for supplying oxygen to reservoir layer 152. For example, reservoir layer 152 may comprise oxygen carriers and absorb oxygen from vasculature surrounding layer 152.

In some applications, reservoir layer 152 is pre-loaded with oxygen carriers (e.g., hemoglobin-based oxygen carriers such as chemically-modified hemoglobin, or "microbubbles" which comprise fluorocarbons such as dodecafluoropentane or perfluorodecalin). Typically, the oxygen carriers are loaded with oxygen and are supplied to reservoir layer 152 at regular intervals following the implantation of system 150.

Each layer 32 of cells has (a) an interface 30 with tissue of subject 22, and (b) an interface with reservoir layer 152 which functions as oxygen-delivery interface 27. In such applications, system 150 functions as housing 24 or 124. Thus, interfaces 30 of each layer 32 with tissue of subject 22 function as oxygen delivery interfaces 27 which absorb oxygen from vasculature surrounding system 150. In such applications, either surface of each layer 32 is exposed to oxygen. That is, the surface of each layer 32 at interface 30 receives oxygen from vasculature of subject 22, while the surface of each layer at the interface between layer 32 and reservoir layer 152 receives oxygen from reservoir layer 152.

It is to be noted that although only two layers 32 of cells are shown, any suitable number of layers of cells may be coupled to reservoir layer 152. It is to be further noted that layers 32 are shown as being flat by way of illustration and not limitation. For example, layer 32 may be shaped to define various thicknesses along the slab. In some applications, layer 32 is thicker at the center and thinner along the edges.

In some applications, system 150 is integrated within housing 24 (described hereinabove with reference to Figs. 1A-B and 2-3, and as described hereinbelow with reference to Figs. 5A-C) or with housing 124 as described hereinbelow with reference to Figs. 6-7. For applications in which system 150 is integrated within housings 24 or 124, interface 30 of a first layer 32 is in contact with tissue of subject 22, while interface 30 of a second layer 32 is in contact with reservoir 42 of housing 24. In such applications, interface 30 that is in contact with tissue of subject 22 functions as oxygen delivery interface 27, because interface 30 facilitates transport of oxygen to the islets in layer 32 from surrounding vasculature of subject 22.

Typically, layer 152 has a diameter of around 10 cm and a thickness of between 1 mm and 3 cm, e.g., around 2 mm, while each layer 32 of islets has a smaller diameter of around 8 cm and a thickness of less than 1 mm. The larger diameter of reservoir layer 152 with respect to layers 32 of islets create portions of layer 152 that are exposed to tissue of subject 22. These portions create a greater surface area for reservoir layer to absorb oxygen from surrounding vasculature of subject 22. For applications in which reservoir layer comprises the oxygen carriers, the oxygen carriers absorb the excess oxygen in layer 152.

Fig. 4B shows a cross-section of oxygen reservoir layer 152. Layer 152 comprises walls 153 comprising a flexible material, e.g., silicone. Walls 153 define a series of channels 157 for directed transport of oxygen through layer 152. Typically, layer 152 comprises unidirectional valves, e.g., mechanical or electromechanical valves, which facilitate directional transport of the oxygen-containing fluids within channels

157. Typically, the fluid transport is induced passively in response to increased pressure within layer 152 in response to an increase of oxygen in layer 152. For some applications, system 150 is implanted in the ribcage of subject 22. In such applications, the fluid within layer 152 is circulated in response to movements of layer 152 responsively to the movements of the ribcage during respiration.

It is to be noted that the number and spatial configurations of walls 153 and valves 155 are shown by way of illustration and not limitation, and that layer 152 may comprise any suitable number of valves and walls which direct the flow of oxygen in any suitable direction. Walls 153 may be oriented in a manner which provides channels 157 shaped differently than as illustrated.

Figs. 5A-C show a system 130 comprising subcutaneously-implantable housing 24 which is configured to receive oxygen from a trachea 100 of subject 22. Housing 24 is typically disposed remotely from trachea 100, and is coupled thereto via an air-transport tube 132. Typically, housing 24 is disposed in the abdomen of subject 22. For some applications, housing 24 is disposed in the ribcage of subject 22.

Transport tube 132 typically comprises silicone and is coupled at a first end 134 thereof to housing 24 and is thereby in fluid communication with reservoir 42 (as shown in Figs. 5A-B). A second end of tube 132 is coupled to a base of a "T"-shaped tracheal mount 136 (as shown in Fig. SC). Respective ends 142 and 144 of tracheal mount 136 are disposed within trachea 100 of subject 22. Ends 142 and 144 define ends of a vertical lumen of tracheal mount 136 and function as a shunt to channel air toward tube 132 such that the channeled air eventually reaches housing 24. As such, tracheal mount 136 functions as oxygen delivery interface 27.

Housing 24 comprises scaffold 25 which supports upper surface 31. In the applications shown in Figs. 5A-B, upper surface 31 is flexible, and depressible. Housing 24 is subcutaneously implanted such that surface 31 i$ depressible by subject 22 when force is applied to surface 31 in response to subject 22 pressing on the skin above surface 31. As shown in Fig. 5A, responsively to the pressing, air is forced out of
reservoir 42 (as indicated by the arrow), through tube 132, and is emptied into trachea 100 via tracheal mount 136.

Fig. 5B shows the release of surface 31 following the pressing thereof since surface 31 is resilient, surface 31 returns to its original shape, as shown, which creates lower pressure in housing 24 and forces oxygen-containing air from trachea 100 into reservoir 42 of housing 24 (as indicated by the arrow). Thus, system 130 functions as a pumping mechanism which enables subject 22 to pump oxygen-containing air into housing 24 from trachea 100.

Reference is now made to Figs. 6-7, which are schematic illustrations of a system 120 comprising a subcutaneously-implantable housing 124 in fluid communication with and coupled to a transcutaneous fluid inlet tube 60 and a transcutaneous fluid outlet tube 62. Respective first ends 64 and 66 of tubes 60 and 62 are disposed within housing 124, while respective second ends 61 and 63 of tubes 60 and 62 are disposed externally to skin 26 of the subject. End 61 of fluid inlet tube 60 serves as oxygen delivery interface 27. Respective plugs 80 are coupled to each end 61 and 63 of tubes 60 and 62, respectively, and function to reversibly seal tubes 60 and 62 when the tubes are not in use.

It is to be noted that tubes 60 and 62 are disposed at the same side of housing 124 by way of illustration and not limitation. For example, tubes 60 and 62 may be disposed at opposite sides of housing 124.

Housing 124 comprises a scaffold 125, as described hereinabove with respect to scaffold 25 with reference to Figs. 1A-B. Scaffold 125 surrounds reservoir 42 and supports one or more layers 32 of functional cells. Typically, one layer 32 is disposed at an upper surface of housing 124 and another layer 32 is disposed at a lower surface of housing 124. A respective gas-permeable membrane 35 is disposed between each layer 32 of .functional cells and reservoir 42. For applications in which housing 124 holds 400,000 islets, each layer 32 of functional cells comprises 200,000 islets. For applications in which housing 124 holds 40,000 islets, each layer 32 of functional cells comprises 20,000 islets.

A source of oxygen (not shown), e.g., a vessel such as a syringe or a pre-filled cartridge, comprising a source of oxygen-containing fluid is coupled to tube 61 at oxygen delivery interface 27. The source of oxygen supplies the fluid to housing 124 via fluid inlet tube 61. In some applications, the source of oxygen comprises air or another mixture of gases. Alternatively, the source of oxygen comprises pure oxygen. In some applications, the source of oxygen comprises a liquid comprising oxygen carriers, as described hereinabove. In conjunction with supplying oxygen to housing 124, fluid having a low oxygen content is expelled from within housing 124 via fluid outlet tube 62. In some applications, the fluid is passively expelled from housing 124 in response to pressure introduced within housing 124 due to the injection of the fluid having a high oxygen content. Alternatively or additionally, a source of suction is coupled at end 63 of tube 62 and actively draws fluid from within housing 124.

As shown, first end 64 of tube 60 is disposed within housing 124 at a substantial distance from end 66 of tube 62 (e.g., greater than 50% of the longest dimension of housing 124, or greater than 75% of the longest dimension of housing 124). Such a configuration allows the transport of fluid from the source of oxygen to housing 124 via fluid inlet tube 60, while minimizing the possibility of immediate withdrawing of the transported fluid through fluid outlet tube 62.

A moisture-absorbing element (not shown) is disposed in housing 124 in a vicinity of end 66 of fluid outlet tube 62 or elsewhere in the housing. The moisture absorbing element helps facilitate a balance of moisture in housing 124. Housing 24 (described hereinabove with reference to Figs. 1A-B) may also comprise the moisture-absorbing element.

Reservoir 42 of housing 24 comprises foam, e.g., an open- cell silicone foam, to maintain a distance between and support layers 32 of functional cells (in a manner similar to supports 36 of housing 24 described hereinabove with reference to Figs. 1A-B). Alternatively or additionally, supports 36 are integrated into housing 124.

Reference is now made to Figs. 1A-B, and 2-7. Following the replenishing of housings 24 and 124 with fluid having a high oxygen content, the source of oxygen is detached from oxygen delivery interface 27. At regular intervals, e.g., once every 1-7 days or once every 7-35 days, the source of oxygen is coupled by the user to interface 27 and supplies oxygen to housing 24 and 124.

Reference is now made to Figs. 3, 4, 5A-C, and 6-7. Reservoir layer 152 of system 150 (described hereinabove with reference to Fig. 4) may be used in combination with any of oxygen delivery interfaces 27 described hereinabove with reference to Figs. 3, 5A-C, and 6-7. That is, in some applications, reservoir layer 152 is coupled to tubes
170 and is loaded with oxygen via ports 172 that are coupled to tubes 170, in a manner as described hereinabove with reference to Fig. 3. For some applications, reservoir layer 152 is coupled to tube 132 which receives oxygen-containing gas from trachea 100, in a manner as described hereinabove with reference to Figs. 5A-C. In some applications, reservoir layer 152 is coupled to transcutaneous tubes 60 and 62 and receives oxygen-containing fluid from fluid-inlet tube 60, in a manner as described hereinabove with reference to Figs. 6-7.

Typically, layers 32 and 152 of system 150 are flexible and are configured for implantation in a vicinity of a ribcage of subject 22. In such applications, reservoir layer 152 moves and contorts responsively to the natural movements of the ribcage of subject 22. Consequently, the oxygen-containing fluid disposed within reservoir layer 152 is forcefully moved therein such that the fluid is circulated within layer 152 and toward the surrounding layers 32 of islets. In some applications, layer 152 comprises a liquid or gel, e.g., alginate. For applications in which layer 152 comprises a gel, layer 152 may be shaped to define channels and or valves which create a path for directed transport of the oxygen-containing fluid within layer 152.

Reference is yet again made to Figs. 1A-B and 2-7. Apparatus described herein may comprise layers 32 of functional cells independently of or in combination with grids 162 and 164 and/or membranes 35 and 166, as described hereinabove with reference to Fig. 2.

Reference is yet again made to Figs. 1A-B and 2-7. It is to be noted that the scope of the present invention includes the use of apparatus described herein for implanting functional cells other than pancreatic islets of Langerhans, e.g., cells of the thyroid gland, cells of the adrenal gland, hepatic cells, and cells that are genetically modified so as to secrete a therapeutic protein.

Reference is additionally made to Figs. 1A-B and 2-7. In some applications, housings 24 and 124 are coupled to a subcutaneously-implantable source of oxygen, or reservoir. The implantable oxygen source is coupled to housings 24 and 124 via at least one tube. For some applications, oxygen is passed from the oxygen source to housings 24 and 124 in an at least somewhat regulated manner. For example, the tube connecting the implanted oxygen source to housing 24 or 124 may comprise a unidirectional valve (e.g., a mechanical or electromechanical valve) which helps regulate a rate of transport of fluid into housing 24 or 124. Typically, the implanted source of oxygen comprises between 100 ml and 300 ml of fluid at a pressure of up to 1000 atm (e.g., 1-10 atm, 10-100 atm, or 100-1000 atm).

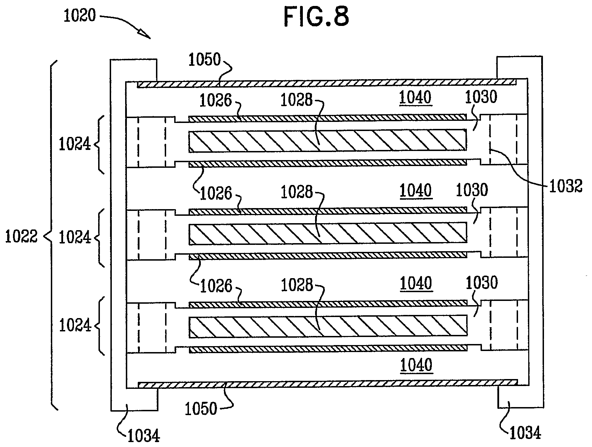

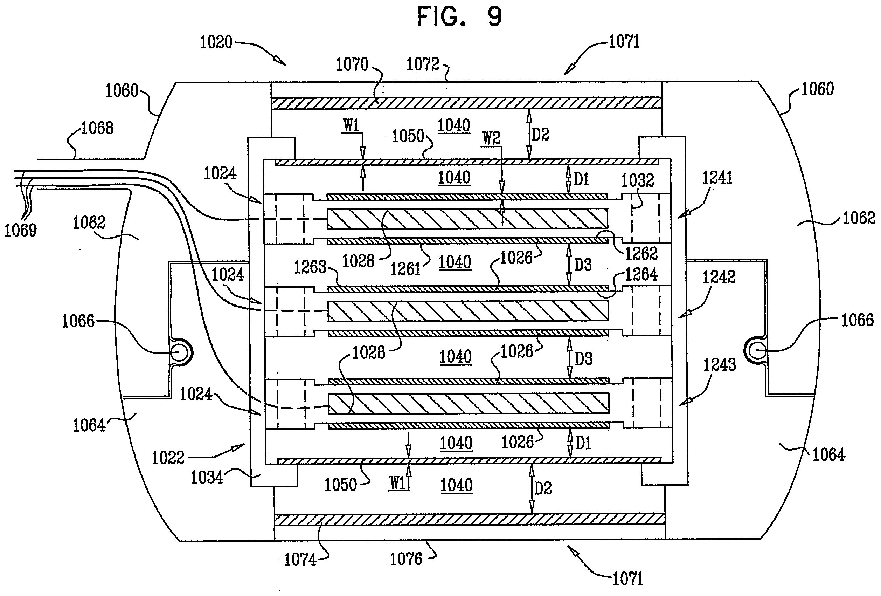

Reference is now made to Figs. 8 and 9, which are schematic illustrations of a system 1020 comprising an oxygen generator 1022 configured to generate and supply oxygen to respective first and second layers 1070 and 1074 of functional cells, in accordance with some applications of the present invention. Typically, the functional cells disposed within layers 1070 and 1074 comprise pancreatic islets of Langerhans cells. Layers 1070 and 1074 of functional cells comprise a metal grid (not shown) which houses a substrate to which the cells adhere, e.g., a liquid or gel, such as alginate, agarose, or polyethylene glycol (PEG). Alternatively, layers 1070 and 1074 are shaped to provide a three-dimensional biodegradable or non-biodegradable fibrillar matrix upon which the cells are dispersed.

Oxygen generator 1022 and layers 1070 and 1074 of functional cells are disposed within a housing 1060 which is configured for implantation within a body of a subject. Typically, oxygen generator 1022 generates sufficient oxygen to sustain the functional cells during the period preceding vascularization of fibrotic tissue that forms around housing 1060. The immediate supply of oxygen to the cells enables proper function of the cells immediately following implantation. Typically, oxygen generator 1022 generates oxygen in real-time by photosynthesis. For some applications, the oxygen generator continues to provide oxygen to the cells during the period following the vascularization of the fibrotic tissue.

Fig. 8 shows oxygen generator 1022 comprising an oxygen supply housing 1034 which, in turn, holds at least one photosynthetic oxygen supply 1024. Each photosynthetic oxygen supply 1024, in turn, comprises a light source 1028, e.g., at least one LED coupled to a light-propagating means such as an illuminating panel, and at least one layer 1026 of algae disposed around light source 1028. As shown, layers 1026 of algae are disposed adjacently to light source 1028 and at a suitable distance therefrom. In some applications, layers 1026 contact light source 1028 (configuration not shown).

In some applications, light source 1028 has an input electrical power between 5 mW/(cm^2 of algae) and 50 mW/(cm^2 of algae). Typically, an energy of illumination applied to layers 26 of the algae is at a rate of between 0.2 mW/(cm^2 of algae) and 2.0 mW/(cm^2 of algae).

In some applications of the present invention, oxygen generator 1022 comprises between one and four (e.g., typically, two) layers of photosynthetic oxygen supplies 1024. A respective air gap 1040 is disposed in fluid communication with layers 1026 of algae of each photosynthetic oxygen supply 1024 and between oxygen generator 1022 and layers 1070 and 1074 of functional cells. The layers of photosynthetic oxygen supplies 1024 each comprise one or more layers 1026 of algae. Typically, layers 1026 of algae provide a large surface area in which a ratio of the surface area of the algae to the surface area of layers 1070 and 1074 of functional cells is greater than 1:1.

Air gaps 1040 of system 1020 provide a means by which oxygen molecules permeate through a gaseous medium with minimal resistance from an area of high concentration (i.e., layers 1026 of algae) to an area of low concentration (i.e., layers 1070 and 1074 of functional cells).

As shown, each oxygen supply 1024 comprises a scaffold 1030 which houses light source 1028 and the algae. Scaffold 1030 comprises a substrate, e.g., agarose or optically-transparent plastic, surrounding light source 1028. Typically, a first and a second layer 1026 of algae are disposed upon scaffold 1030 adjacent to respective first and second surfaces of light source 1028. Such disposition of layers 1026 with respect to light source 1028 increases a surface area of algae exposed to light emitted from source 1028, effectively increasing a rate of photosynthesis of the algae.

In some applications of the present invention, light source 1028 comprises a plurality of LEDs coupled to a plastic illuminating panel which conducts the light emitted from the LEDs.

Reference is again made to Figs. 8 and 9. Each photosynthetic oxygen supply 1024 is separated from the next oxygen supply 1024 by an air gap 1040 comprising a gas, e.g., air enhanced by oxygen and carbon dioxide. The gas is disposed in air gap 1040 between adjacent oxygen supplies 1024 such that each layer 1026 of algae is in fluid communication with the gas, and oxygen generated by the algae is diffused into air gap 1040. The presence of the gas disposed within air gap 1040 is hypothesized by the inventors to increase the diffusivity of the oxygen through air gap 1040 and ultimately toward layers 1070 and 1074 of functional cells. The gas disposed within air gap 1040 also enables storage therein of a large amount of gaseous oxygen produced from the algae. As shown in Fig. 9, each air gap 1040 between respective oxygen supplies 1024 (which, in the context of Fig. 9, are oxygen supplies 1241, 1242, and 1243) is shaped to define a distance D3 of between 100 um and 300 um, e.g., 200 um.

Scaffolds 1030 are shaped to provide at least one channel 1032 configured for passage therethrough of the oxygen generated by oxygen supplies 1024 to layers 1070 and 1074 of functional cells. As shown in the applications of the figures, channels 32 facilitate continuous gas communication among the various air gaps 1040. Since the functional cells consume oxygen at a variable rate depending on local conditions (e.g., blood glucose level), and since the algae in layers 1026 produce oxygen at a variable rate depending on local conditions (e.g., light intensity levels), air gaps 1040 function as oxygen reservoirs storing the generated oxygen until the oxygen is diffused upon need therefor to the functional cells. Such storing regulates a concentration of oxygen within housing 1060.

Thus, when light is provided at a constant rate, the algae produce oxygen at a constant rate, all other things being equal. The oxygen is then diffused into air gaps 1040 where, depending on the need for oxygen consumption by the functional cells, the oxygen molecules may: (1) diffuse through air gaps 1040 and toward the functional cells where it is used immediately, (2) be stored in air gaps 1040 until there exists a demand for oxygen by the functional cells, or (3) once air gaps 1040 are saturated with oxygen and when the functional cells do not need or consume the available oxygen stored in air gaps 1040, permeate through cell layers 1070 and 1074, through membranes 1072 and 1076, respectively, and toward surrounding tissue of the subject.

Additionally, a light regulator (not shown) is typically coupled to housing 1060, to regulate the concentration of oxygen in housing 1060 by regulating the rate of production of oxygen by the algae. The light regulator typically regulates the intensity of light emitted from light source 1028 of each oxygen supply 1024, or a pulse rate or a duty cycle of the emitted light.

As shown in Fig. 9, each layer 1026 of algae is shaped to define a width W2 of between 10 um and 2000 um, typically, between 100 um and 2000 um, e.g., between 100 um and 300 um. Additionally, each layer 1026 of algae is disposed substantially in a planar configuration defining dimensions of between about 0.05 cm by 0.05 cm and about 10 cm by 10 cm (e.g., 1.3 cm by 1.8 cm).

Fig. 9 shows oxygen generator 1022 comprising oxygen supplies 1241, 1242, and 1243. Width W2 of each layer 1026 of algae in each oxygen supply 1024: (a) optimizes the propagation of light from light source 1028 within layer 26, (b) reduces the relative oxygen concentration disposed within layers 1026, and (c) facilitates a proper oxygen flux between layers 1026 and air gaps 1040. Width W2 of algae layers 1026 enables most of the algae to absorb generally around 90% of light emitted from light source 1028.

Additionally, width W2 of layer 1026 facilitates transmission of light to algae layer 1026 of a first oxygen supply 1024 from a neighboring light source 1028 of a second oxygen supply 1024. This is because the small width of algae layer 1026 disposed adjacently to light source 1028 of the second oxygen supply facilitates transmission of light through layer 1026 of algae and toward algae layer 1026 of the first oxygen supply. For example, due to a relatively thin width W2 of algae layer 1026, sufficient light generated by light source 1028 of oxygen supply 1241 is able to propagate beyond surface 1261 of algae layer 1026 of oxygen supply 1241, and toward surface 1263 of algae layer 1026 of oxygen supply 1242. Additionally, surface 1263 of algae layer 1026 of oxygen supply 1242 also receives light that is generated by light source 1028 of oxygen supply 1242 because, due to the relatively thin width W2 of algae layer 1026, light is able to propagate through (and beyond) algae layer 1026.

Thus, the light intensity, or irradiance, at a given surface of a given algae layer 1026 (i.e., the surface that is disposed in optical communication with the light source of a neighboring oxygen supply) is typically a combination of intensities of: (1) light that is emitted from the adjacently-disposed light source, and (2) light that is emitted from the neighboring light source.

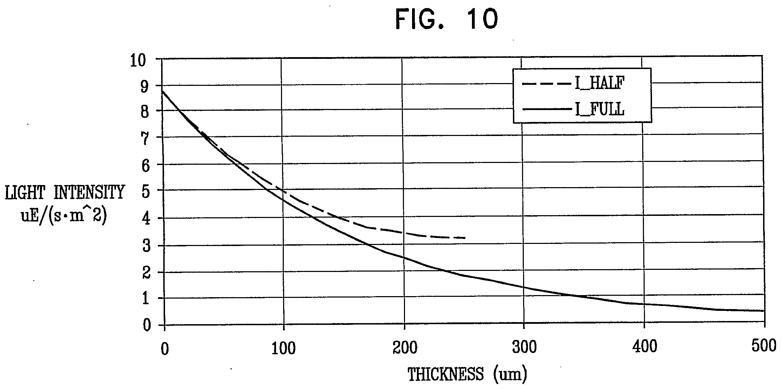

Reference is now made to Figs. 10-12, which are graphical representations of parameters of the oxygen generator.

Fig. 10 is a graphical illustration of a comparison of light intensity at various widths (i.e., 250 um and 500 um) of algae layers of respective, adjacently-disposed photosynthetic oxygen supplies. Typically, according to the Beer-Lambert law, light decays exponentially (as shown) through the respective algae layers which have a full width (i.e., 500 um) and have a width that is half the full width (i.e., 250 um). The graph represents the algae layers in which: (a) 0 um defines the surface of the algae layer that is disposed adjacently to the light source, and (b) 250 um or 500 um defines the surface of the respective algae layer that is furthest away from the light source.

Thus, for the algae layer having a width of 500 um, light intensity decays from around 8.8 uE/(s*m^2) at 0 um to around 0.3 uE/(s*M^2) at 500 um, i.e., the algae layer surface furthest from the light source. In contrast, for the algae layer having a width of 250 um, light intensity decays from around 8.8 uE/(s*m^2) at 0 um to around 3.1 uE/(s*m^2) at 250 um, i.e., the algae layer surface furthest from the light source. As described hereinabove with reference to Fig. 9, by reducing the width of the algae layer 26 to around 250 um, light is able to pass through the algae layer of a first oxygen supply and toward the algae layer of a second, neighboring oxygen supply, in order to supplement light to the neighboring layer which also receives light from a light source disposed adjacently thereto. This supplemented light at the surface of the 250 um-wide algae layer increases the light intensity at the surface relative to the light intensity at the surface of a 500 um-wide algae layer.

Although the light intensity has decayed at 250 um from the light source in either curve, the curve representing the 250 um-wide algae layer shows a relatively stronger light intensity at the surface of the 250 um-wide algae layer (i.e., the surface exposed to and in fluid communication with the air gap). This is because, as described hereinabove with respect to Fig. 9, width W2 of layer 1026 of algae enables the algae at 250 um from the light source to receive light from the light source disposed adjacently to the algae layer as well as from the light source of a neighboring photosynthetic oxygen supply.

The light intensity at the surface of the 250 um-wide algae layer optimizes the total oxygen production of the.entire algae layer since the algae at the surface of the 250 um-wide layer are provided with sufficient light to undergo photosynthesis more efficiently than the algae at the surface of the 500 um-wide algae layer.

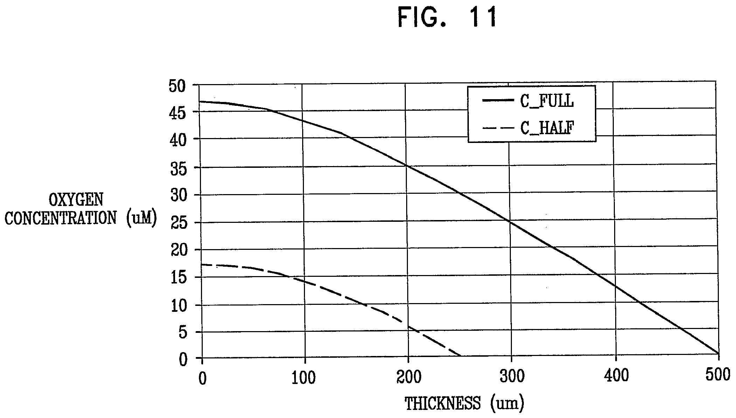

Fig. 11 shows relative oxygen concentrations disposed within the 250 um-wide algae layer and within the 500 um-wide algae layer. The graph represents the algae layers in which: (a) 0 um defines the surface of the algae layer that is disposed adjacently to the light source, and (b) 250 um or 500 um defines the surface of the respective algae layer that is furthest away from the light source. Typically, the concentration of oxygen in either algae layer is greatest at 0 um due to the proximity of the algae to the light source.

Reference is now made to Figs. 10, 11, and 12, which are graphical representations of calculated, simulated parameters of the oxygen generator, in accordance with some applications of the present invention. As described hereinabove with reference to Fig. 10, the algae at the surface of the 250 um-wide layer that is exposed to the air gap receives sufficient light in order to produce oxygen. This oxygen is immediately diffused into the air gap. Additionally, the oxygen produced by the algae layer at 0 um diffuses a relatively shorter distance through the 250 um-wide algae layer than through the 500 um-wide algae layer. Thus, as indicated in Fig. 11, the concentration of oxygen deep in the 250 um-wide algae layer (i.e., ∼17 uM) is lower than the concentration of oxygen deep in the 500 um-wide algae layer (i.e., ~48 uM). The 250 um-wide algae layer facilitates enhanced diffusion of oxygen molecules therethrough in order to: (a) reduce the oxygen toxicity of the algae layer (in particular of algae disposed adjacently to light source 1028 in surfaces 1262 and 1264, for example), and (b) diffuse more oxygen molecules into air gap 1040.

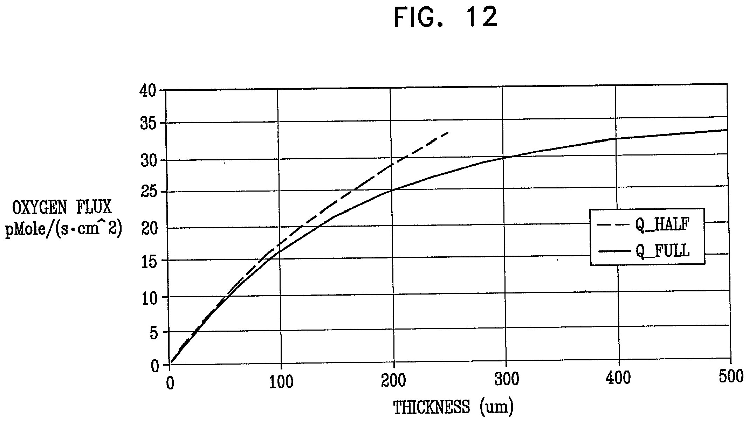

Fig. 12 shows the oxygen flux for the respective algae layers. As shown, the 250 um-wide and 500 um-wide algae layers both generate substantially equal oxygen fluxes (i.e., 34 pMoles/(s*cm^2)) through their exposed surfaces. Taken together with the information of the graphs with reference to Figs. 10 and 11, the 250 um-wide algae layer provides an oxygen flux that is substantially equal to the oxygen flux of the 500 um-wide algae layer because: (1) the width of the 250 um-wide algae layer provides a shorter distance for oxygen diffusion toward the air gap, which reduces the oxygen concentration within the algae layer, while (2) the width of the 500 um-wide algae layer provides a longer distance for diffusion of the oxygen through the algae layer which effectively retains the oxygen within the algae layer. Thus, the air gaps in device 1020 facilitate oxygen provision to the functional cells while reducing the overall footprint of the device, providing reservoirs for excess oxygen molecules, and facilitating a more effective utilization of the algae in the entire layer.

For applications in which oxygen generator 1022 of device 1020 comprises only one photosynthetic oxygen supply 1024, air gaps 1040 function provide oxygen reservoirs for excess oxygen molecules and facilitate a more effective utilization of the algae in the entire layer.

For some applications, scaffolds 1030 are configured to comprise minerals and/or other substances (e.g., potassium nitrate, sodium chloride, sodium phosphate, and/or a magnesium compound) which regulate a level of humidity within housing 1060. Such regulation is effected by controlling the osmotic pressure of water vapor within air gaps 1040. Typically, when osmotic pressure is elevated beyond a threshold amount, water vapor condenses. To ensure a level of relative humidity below 100% within housing 1060, a concentration of minerals for each scaffold 1030 is determined during production of scaffolds 1030 such that the minerals will sufficiently absorb the condensed water vapor within air gaps 1040 once housing 1060 is implanted within the body of the subject. In turn, by reducing the level of humidity within housing 1060, the minerals disposed within scaffolds 1030 reduce the osmotic pressure in housing 1060. The concentration of minerals in scaffolds 1030 is chosen such that the minerals will absorb just enough condensed water to: (a) reduce a level of humidity in housing 1060, and (b) normalize the osmotic pressure so as to prevent liquid from being absorbed into housing 1060 (from a site outside housing 1060), should the minerals create a hypo-osmotic environment within housing 1060 by absorbing too much water. The appropriate concentration of minerals in scaffolds 1030 absorbs condensed water vapor sufficiently such that the algae-growth conditions are not compromised, thereby maintaining a consistent level of moisture within layers 1026 of algae.

In some applications, in order to prevent drying of the algae, light source 1028 is positioned at a distance of 0.1 mm and 2.0 mm away from layers 1026 of algae. In such applications, light is generated remotely by light source 1028 and is conducted toward the layers of algae via an illuminating panel, via an optically-transparent tube, or via an optical fiber.

In some applications, scaffolds 1030 comprise a material configured to incrementally secrete the minerals over a period of time. In such applications, the material helps regulate a level of minerals in housing 1060 at a given time and helps prevent the algae from being dried out (an effect of an unregulated, continuous presence of a high concentration of minerals). Furthermore, the algae typically consume a portion of the minerals which regulate the osmotic pressure in housing 1060. As such, at given intervals, the material which secretes the minerals provides to scaffolds 1030 a renewed supply of minerals in the absence thereof. In some applications, the secretion of minerals by the material is governed by an open-loop controller which is not responsive to sensed parameters within housing 1060. In some applications, the secretion of minerals by the material is governed by a closed-loop controller, e.g., a PID controller. In such applications, scaffolds 1030 comprise a sensor configured to sense a parameter within housing 1060, e.g., osmotic pressure and/or condensation/humidity. The material is then actuated by the controller to secrete the minerals in response to a signal generated by the sensor when the sensor detects an elevated level of humidity and/or osmotic pressure.

Reference is again made to Fig. 9, which is a schematic illustration of a disposition of oxygen generator 1022 with respect to housing 1060. Housing 1060 is typically shaped to provide an upper portion 1062 and a lower portion 1064 of housing 1060. Upper and lower portions 1062 and 1064 each comprise a respective first and second layer 1070 and 1074 of functional cells. Prior to implantation of housing 1060 in the body of the subject, housing 1060 is loaded with oxygen generator 1022 such that oxygen generator 1022 is disposed between first and second layers 1070 and 1074 of functional cells. Upper and lower portions 1062 and 1064 of housing 1060 are aligned at least in part to surround oxygen generator 1022. An O-ring 1066 is disposed within a groove between portions 1062 and 1064, and then upper and lower portions 1062 and 1064 are sealed together. O-ring 1066 is used as a mechanical pressure seal at an interface between portions 1062 and 1064. Typically, O-ring 1066 functions to maintain the pressure within housing 1060 and to prevent leakage of oxygen from within housing 1060.

Housing 1060 comprises an electrical channel 1068 which couples housing 1060 to a remote power supply (not shown). Typically, the power supply is implanted within the body of the subject in the vicinity of housing 1060. Alternatively, the power supply is disposed outside the body of the subject, e.g., on an article of clothing such as a belt, and channel 1068 transdermally. couples the power supply to housing 1060. Channel 1068 houses electrical wires 1069, and each electrical wire 1069 supplies power to a respective light source 1028 of oxygen generator 1022.

Oxygen supply housing 1034 is shaped to provide at least one gas-permeable membrane 1050 configured for diffusion therethrough of the oxygen toward layers 1070 and 1074 of functional cells. A respective gas-permeable membrane 1050 is typically disposed between oxygen generator 1022 and layers 1070 and 1074 of functional cells. Each gas-permeable membrane 1050 is typically shaped to define: (a) a width W1 of between 5 um and 1000 um, e.g., between 10 um and 100 um, and (b) a diameter of between 1 cm and 25 cm, e.g., 3.5 cm. Gas-permeable membrane 1050 is spaced apart from respective first and second layers 1070 and 1074 of functional cells by an air gap 1040 defining a distance D2. Distance D2 typically ranges from about 1 um to about 1000 um, such as 200 - 500 um (e.g., 300 um). Gas-permeable membrane 1050 is, in turn, spaced apart from a layer 1026 of algae by an air gap 1040 having a distance D1 of about 0.1 mm to about 6 mm, e.g., 0.5 mm. As described hereinabove, gas is disposed within each air gap 1040 between respective layers of housing 1060.

Housing 1060 typically comprises a selectively-permeable biocompatible membrane 1071 encapsulating either the entire housing, or portions thereof (e.g., covering at least layers 1070 and 1074 of cells, as shown). Membrane 1071 comprises a first membrane 1072 which is disposed adjacent to layer 1070 of cells, and comprises a second membrane 1076 which is disposed adjacent to layer 1074 of cells. Membrane 1071 is typically characterized by a molecular weight cut-off of about 11,000-100,000 daltons, that is adapted to allow passage of nutrients and substances produced or consumed by the cells, such as oxygen, carbon dioxide, glucose, insulin, or water. Membrane 1071 of housing 1060 also typically immunoisolates the functional cells by preventing passage therethrough of cells of the subject's body, e.g., white blood cells.

Additionally, the gas disposed in each air gap 1040 of housing 1060 further immunoisolates system 1020, since a liquid medium typically facilitates cell motility.

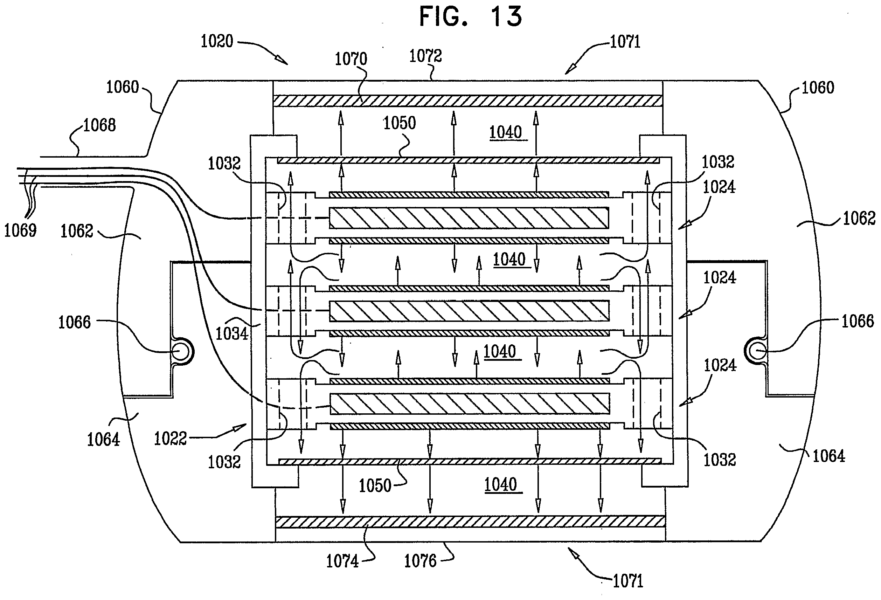

Reference is now made to Fig. 13, which is a schematic illustration of a flow of oxygen within housing 1060, in accordance with some applications of the present invention. The arrows of Fig. 13 represent the flow of oxygen as it diffuses from oxygen generator 1022 toward layers 1070 and 1074 of functional cells. Since each layer 1026 of algae is in contact with air gap 1040 comprising the gas, the oxygen molecules generated in layers 1026 are diffused into air gap 1040. The oxygen can diffuse among and be stored within different air gaps 1040 via channels 1032 until the oxygen is ultimately diffused through respective gas-permeable membranes 1050 toward layers 1070 and 1074 of functional cells. It is to be noted that the use of three layers of oxygen supplies 1024 is shown by way of illustration and not limitation, and that the scope of the present invention includes applications in which between one layer and ten layers, e.g., one layer, of oxygen supplies 1024 are used.

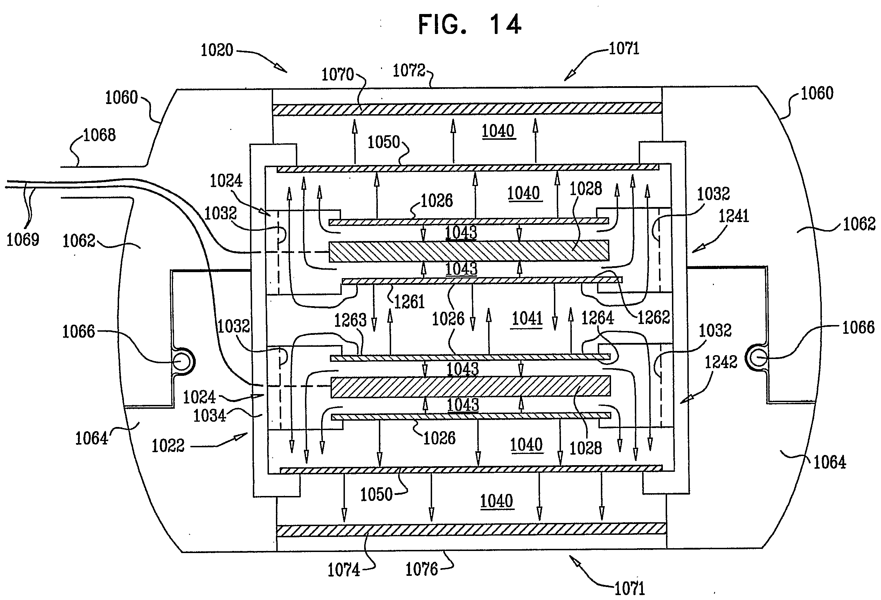

Reference is now made to Fig. 14, which is a schematic illustration of system 1020 comprising oxygen generator 1022 comprising two oxygen supplies 1024, which, in the context of Fig. 14, are oxygen supplies 1241 and 1242, and a plurality of air gaps 1043 disposed between layers 1026 of algae and light source 1028, in accordance with some applications of the present invention. Layers 1026 of algae comprise algae that are disposed within respective gas-permeable membranes. Support 1034 comprises a scaffold that facilitates proper spatial relationship between layers 1026 of membrane-bound algae and light source 1028.

As shown, for each oxygen supply 1024, layers 1026 of algae are coupled to support 1034 in a manner in which an air gap 1043 is disposed between each layer 1026 of algae and light source 1028. In such applications:
(1) a respective air gap 1041 is provided adjacent to each upper surface 1261 and 1263 of algae layer 1026. For example, air gap 1041 is in fluid communication with (a) surface 1261 of algae layer 1026 of supply 1241 that is exposed to air gap 1041, and (b) surface 1263 of layer 1026 of algae that of supply 1242 that is exposed to the same air gap 1041;and
(2) a respective air gap 1043 *is* provided between each layer 1026 of algae and light source 1028. For example, a respective air gap 1043 is disposed between each lower surface 1262 and 1264 of algae layer 1026 and light source 1028.

Air gaps 1041 and 1043 allow the oxygen to diffuse from layers 1026 and into the housing through both surfaces of layer 1026 (i.e., light-remote surfaces 1261 and 1263 and light-adjacent surfaces 1262 and 1264 of each layer 1026), thereby increasing the diffusion of the oxygen from the algae layer. The algae produce most of the oxygen in the vicinity of the layer that is closest to the light source (i.e., light-adjacent surfaces 1262 and 1264). The oxygen from the vicinity is allowed to diffuse into air gap 1043 via light-adjacent surfaces 1262 and 1264 without having to diffuse through algae layer 1026 first before exiting toward air gap 1041 via light-remote surfaces 1261 and 1263.

It is to be appreciated that the ·scope of the present invention includes the oxygenation of transplanted cells other than pancreatic islet cells, such as transplanted cells described in references cited in the Background section of the present patent application. For some applications, vascularization around housing 1060 is enhanced using one or more biochemical factors (e.g., VEGF) coupled to or in a vicinity of housing 1060.

It is to be noted that the scope of the present invention includes the use of device 1020 independently of layers 1070 and 1074 of functional cells. In such applications, device 1020 functions as an oxygen generator and comprises an outlet configured for release of the generated oxygen (application not shown). In some applications, such a device maybe implanted in the body of the subject.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. A system (20), comprising:
a subcutaneously-implantable housing (24), configured for implantation in a body of a subject;
functional cells (32) within the housing;
a source of oxygen disposed outside of the housing for supplying oxygen to the functional cells;
an oxygen delivery interface (27) at an outer surface of the housing for receiving oxygen from the source of oxygen, and for facilitating passage of the oxygen to the functional cells, while the housing is disposed within the body of the subject, the oxygen delivery interface (27) comprising a penetrable surface;
a needle for transcutaneously penetrating a penetrable surface with the needle, the housing (24) being indirectly couplable to the source of oxygen via the needle, and the needle facilitates supplying of oxygen to the functional cells (32) from the source of oxygen,
at least one oxygen-delivery port (172) being disposed remotely from the housing (24) such that a tube (170) coupled at a first end thereof to the port (172) and at a second end thereof to the housing (24), wherein the oxygen-delivery port (172) has an upper surface comprising the penetrable surface of the oxygen delivery interface (27) that is penetrable by the needle, and
an oxygen reservoir (42) within the housing for storing oxygen received from the source of oxygen at a pressure of 1 atm or greater, the oxygen reservoir being in fluid communication with the functional cells.

2. The system according to claim 1, wherein the functional cells comprise cells disposed in pancreatic islets.

3. The system according to claim 1, wherein the source of oxygen comprises a plurality of gases.

4. The system according to claim 1, wherein the oxygen delivery interface is reversibly couplable to the source of oxygen.

5. The system according to claim 1, wherein a volume of the housing is sufficient to sustain the functional cells for a period of between 12 hours and 2 weeks.

6. The system according to claim 1, wherein the housing is implanted in a vicinity of a ribcage of the subject, and wherein the housing circulates the oxygen in the oxygen reservoir in response to movements of the housing responsively to movements of the ribcage of the subject.

7. The system according to claim 1, further comprising a gas-permeable membrane disposed between the oxygen reservoir and the functional cells.

8. The system according to claim 1, wherein:
the needle comprises at least a first chamber and a second chamber,
the first chamber facilitates delivery of oxygen from the source of oxygen to the functional cells within the housing, and
the second chamber facilitates passage of fluid from within the housing to outside the body of the subject.

9. A method for use with a system according to any one of claims 1 to 8, the method comprising:
at a first time:
coupling a source of oxygen to the interface;
supplying oxygen from the source of oxygen to the functional cells; and
decoupling the source of oxygen from the interface; and
at a second time:
coupling the source of oxygen to the interface;
supplying oxygen from the source of oxygen to the functional cells; and
decoupling the source of oxygen from the interface.

## Patentansprüche

1. System (20), aufweisend:
ein subkutan implantierbares Gehäuse (24), das dafür ausgelegt ist, in den Körper einer Person implantatiert zu werden;
funktionelle Zellen (32) in dem Gehäuse;
eine Sauerstoffquelle, die außerhalb des Gehäuses angeordnet ist, um den funktionellen Zellen Sauerstoff zuzuführen;
eine Sauerstoffzufuhrschnittstelle (27) an einer Außenfläche des Gehäuses zum Aufnehmen von Sauerstoff von der Sauerstoffquelle und zur Ermöglichung eines Stroms des Sauerstoffs in die funktionellen Zellen, während das Gehäuse in dem Körper der Person angeordnet ist, wobei die Sauerstoffzufuhrschnittstelle (27) eine durchdringbare Fläche aufweist;
eine Nadel zum transkutanen Durchdringen einer durchdringbaren Fläche mittels der Nadel, wobei das Gehäuse (24) über die Nadel indirekt an die Sauerstoffquelle ankoppelbar ist, und die Nadel ein Zuführen von Sauerstoff von der Sauerstoffquelle in die funktionellen Zellen (32) ermöglicht,
wenigstens einen Sauerstoffzufuhranschluss (172), der entfernt von dem Gehäuse (24) angeordnet ist, so dass ein Rohr (170)an einem ersten Ende davon mit dem Anschluss (172) und an einem zweiten Ende davon mit dem Gehäuse (24) gekoppelt ist, wobei der Sauerstoffzufuhranschluss (172) eine Oberseite hat, die die durchdringbare Fläche der Sauerstoffzufuhrschnittstelle (27) aufweist, die mittels der Nadel durchdringbar ist, und
einen Sauerstoffbehälter (42) im Inneren des Gehäuses zum Speichern von Sauerstoff, der von der Sauerstoffquelle bei einem Druck zwischen 1 atm oder darüber aufgenommen ist, wobei der Sauerstoffbehälter mit den funktionellen Zellen in Fluidaustausch verbunden ist.

2. System nach Anspruch 1, wobei die funktionellen Zellen Zellen beinhalten, die in Pankreasinseln angeordnet sind.

3. System nach Anspruch 1, wobei die Sauerstoffquelle mehrere Gase enthält.

4. System nach Anspruch 1, wobei die Sauerstoffzufuhrschnittstelle mit der Sauerstoffquelle reversibel ankoppelbar ist.

5. System nach Anspruch 1, wobei ein Volumen des Gehäuses dafür geeignet ist, die funktionellen Zellen für einen Zeitraum von zwischen 12 Stunden und 2 Wochen aufrecht zu erhalten.

6. System nach Anspruch 1, wobei das Gehäuse in der Nähe des Brustkorbs der Person implantiert ist, und wobei das Gehäuse den Sauerstoff in dem Sauerstoffbehälter in Reaktion auf Bewegungen des Gehäuses in Abhängigkeit von Bewegungen des Brustkorbs der Person zirkulieren lässt.

7. System nach Anspruch 1, ferner mit einer gasdurchlässigen Membrane, die zwischen dem Sauerstoffbehälter und den funktionellen Zellen angeordnet ist.

8. System nach Anspruch 1, wobei:
die Nadel wenigstens eine erste Kammer und eine zweite Kammer aufweist,
die erste Kammer eine Zufuhr von Sauerstoff von der Sauerstoffquelle in die funktionellen Zellen in dem Gehäuse ermöglicht, und
die zweite Kammer einen Durchfluss von Fluid aus dem Inneren des Gehäuses zur Außenseite des Körpers der Person ermöglicht.

9. Verfahren zur Verwendung in einem System nach einem beliebigen der Ansprüche 1 bis 8, wobei das Verfahren Folgendes umfasst:
zu einer ersten Zeit:
Ankoppeln einer Sauerstoffquelle an die Schnittstelle;
Zuführen von Sauerstoff von der Sauerstoffquelle in die funktionellen Zellen; und
Abkoppeln der Sauerstoffquelle von der Schnittstelle; und
zu einer zweiten Zeit:
Ankoppeln der Sauerstoffquelle an die Schnittstelle;
Zuführen von Sauerstoff von der Sauerstoffquelle in die funktionellen Zellen; und
Abkoppeln der Sauerstoffquelle von der Schnittstelle.

## Revendications

1. Système (20), comprenant :
un boîtier implantable par voie sous-cutanée (24), configuré pour l'implantation dans un corps d'un sujet ;
des cellules fonctionnelles (32) à l'intérieur du boîtier ;
une source d'oxygène disposée à l'extérieur du boîtier pour administrer de l'oxygène aux cellules fonctionnelles ;
une interface d'administration d'oxygène (27) sur une surface extérieure du boîtier pour recevoir de l'oxygène provenant de la source d'oxygène et pour faciliter le passage de l'oxygène vers les cellules fonctionnelles, pendant que le boîtier est disposé dans le corps du sujet, l'interface d'administration d'oxygène (27) comprenant une surface pénétrable ;
une aiguille pour pénétrer par voie transcutanée une surface pénétrable avec l'aiguille, le boîtier (24) étant couplé indirectement à la source d'oxygène via l'aiguille et l'aiguille facilitant la fourniture en oxygène des cellules fonctionnelles (32) à partir de la source d'oxygène,
au moins un orifice de distribution d'oxygène (172) étant disposé à distance du boîtier (24) de sorte qu'un tube (170) soit couplé à sa première extrémité à l'orifice (172) et à une deuxième extrémité au boîtier (24), dans lequel l'orifice de distribution d'oxygène (172) a une surface supérieure comprenant la surface pénétrable de l'interface d'administration d'oxygène (27) qui est pénétrée par l'aiguille, et
un réservoir d'oxygène (42) à l'intérieur du boîtier pour stocker de l'oxygène reçu de la source d'oxygène à une pression de 1 atm ou plus, le réservoir d'oxygène étant en communication fluidique avec les cellules fonctionnelles.

2. Système selon la revendication 1, dans lequel les cellules fonctionnelles comprennent des cellules disposées dans des îlots pancréatiques.

3. Système selon la revendication 1, dans lequel la source d'oxygène comprend une pluralité de gaz.

4. Système selon la revendication 1, dans lequel l'interface d'administration d'oxygène peut être couplée de manière réversible à la source d'oxygène.

5. Système selon la revendication 1, dans lequel un volume du boîtier est suffisant pour soutenir les cellules fonctionnelles pendant une période comprise entre 12 heures et 2 semaines.

6. Système selon la revendication 1, dans lequel le boîtier est implanté au voisinage d'une cage thoracique du sujet, et dans lequel le boîtier fait circuler l'oxygène dans le réservoir d'oxygène en réponse aux mouvements du boîtier suite aux mouvements de la cage thoracique du sujet.

7. Système selon la revendication 1, comprenant en outre une membrane perméable aux gaz disposée entre le réservoir d'oxygène et les cellules fonctionnelles.

8. Système selon la revendication 1, dans lequel:
l'aiguille comprend au moins une première chambre et une deuxième chambre,
la première chambre facilite la délivrance d'oxygène de la source d'oxygène aux cellules fonctionnelles dans le boîtier, et
la deuxième chambre facilite le passage de fluide depuis l'intérieur du boîtier jusqu'à l'extérieur du corps du sujet.

9. Procédé pour une utilisation avec un système selon l'une quelconque des revendications 1 à 8, le procédé comprenant :
dans un premier temps, les étapes consistant à :
coupler une source d'oxygène à l'interface ;
fournir l'oxygène à partir de la source d'oxygène aux cellules fonctionnelles ; et
découpler la source d'oxygène de l'interface; et
dans un deuxième temps, les étapes consistant à :
coupler la source d'oxygène à l'interface ;
fournir l'oxygène à partir de la source d'oxygène aux cellules fonctionnelles ; et
découpler la source d'oxygène de l'interface.
